# FASCICULE DE BREVET EUROPEEN

(11) **EP 2 819 635 B1**
(45) Date de publication et mention de la délivrance du brevet: **11.07.2018**
(21) Numéro de dépôt: 13711084.7
(22) Date de dépôt: 28.02.2013
(51) Int. Cl.: A61K 8/11, A61K 8/34, A61K 8/42, A61K 8/49, A61K 9/00, A61K 9/14, A61K 9/48, A61K 31/045, A61K 31/198, A61K 31/366, A61K 31/402, A61K 31/473, A61K 45/06, A61K 8/02, A61P 3/04, A61K 36/06, A61K 36/42, A61Q 19/06, A61K 8/43, A61K 8/66, A61K 8/97

(54) **PRODUIT DE COMBINAISON POUR LE TRAITEMENT DU SURPOIDS ET/OU L'AMÉLIORATION DE LA SILHOUETTE**
KOMBINATIONSPRÄPARAT ZUR BEHANDLUNG VON ÜBERGEWICHT UND/ODER ZUR VERBESSERUNG DER FIGUR
COMBINATION PRODUCT FOR TREATING EXCESS WEIGHT AND/OR FOR IMPROVING THE FIGURE

(30) Priorité: 29.02.2012 FR 1251832
(43) Date de publication de la demande: 07.01.2015
(73) Titulaire: Institut National de la Recherche Agronomique (INRA), 75007 Paris (FR); Université Paris Descartes, 75006 Paris (FR); Assistance Publique Hôpitaux De Paris, 75004 Paris 4 (FR)
(72) Inventeur: MORIO-LIONDORE, Béatrice, F-63800 Saint George Sur Allier (FR); CAPEL, Frédéric, F-63540 Romagnat (FR); DE BANDT, Jean-Pascal, F-78290 Croissy Sur Seine (FR); MOINARD, Christophe, 38190 BERNIN (FR); CYNOBER, Luc, F-92330 Sceaux (FR)
(74) Mandataire: Jacobacci Coralis Harle
(86) Numéro de dépôt international: PCT/FR2013/050430
(87) Numéro de publication internationale: WO 2013/128137

(56) Documents cités:
- WO-A1-2004/052368
- WO-A1-2005/115371
- WO-A2-2005/058310
- CN-A- 1 481 796
- CN-A- 101 219 141
- GB-A- 2 199 243
- KR-A- 20030 083 348
- KR-A- 20110 081 672
- US-A1- 2005 288 373
- US-B1- 6 683 116
- DATABASE WPI Week 201048 Thomson Scientific, London, GB; AN 2010-J02244 XP002685337, & CN 101 731 699 A (LIAO C) 16 juin 2010 (2010-06-16)
- DATABASE WPI Week 201156 Thomson Scientific, London, GB; AN 2011-J88570 XP002685338, & CN 102 113 648 A (XU X) 6 juillet 2011 (2011-07-06)
- TAN BIE ET AL: "Dietary L-arginine supplementation differentially regulates expression of lipid-metabolic genes in porcine adipose tissue and skeletal muscle.", THE JOURNAL OF NUTRITIONAL BIOCHEMISTRY MAY 2011 LNKD- PUBMED:20619625, vol. 22, no. 5, mai 2011 (2011-05), pages 441-445, XP002685339, ISSN: 1873-4847
- LI XUE-MEI ET AL: "Advances on the Pharmacological Effects of Red Yeast Rice", CHINESE JOURNAL OF NATURAL MEDICINES, vol. 9, no. 3, mai 2011 (2011-05), pages 161-166, XP002685340, ISSN: 1672-3651
- CYNOBER L ET AL: "Le temps des acides amines est-il revenu ?", NUTRITION CLINIQUE ET METABOLISME, ARNETTE EDITIONS, PARIS, FR, vol. 22, no. 4, 1 décembre 2008 (2008-12-01), pages 135-141, XP025760552, ISSN: 0985-0562, DOI: 10.1016/J.NUPAR.2008.10.002 [extrait le 2008-11-26]
- JOBGEN W S ET AL: "Regulatory role for the arginine-nitric oxide pathway in metabolism of energy substrates", JOURNAL OF NUTRITIONAL BIOCHEMISTRY, BUTTERWORTH PUBLISHERS, STONEHAM, GB, vol. 17, no. 9, 1 septembre 2006 (2006-09-01), pages 571-588, XP024960861, ISSN: 0955-2863, DOI: 10.1016/J.JNUTBIO.2005.12.001 [extrait le 2006-09-01] cité dans la demande
- BERTHE MARIE-CLOTILDE ET AL: "Arginine or citrulline associated with a statin stimulates nitric oxide production in bovine aortic endothelial cells.", EUROPEAN JOURNAL OF PHARMACOLOGY 30 NOV 2011 LNKD- PUBMED:21914450, vol. 670, no. 2-3, 30 novembre 2011 (2011-11-30), pages 566-570, XP002685341, ISSN: 1879-0712

## Description

### DOMAINE DE L'INVENTION :

L'invention est liée au domaine des produits destinés au traitement de la surcharge de masse grasse et/ou du surpoids ou de l'obésité.
L'invention porte également sur des procédés de traitement cosmétique pour réduire ou prévenir l'accumulation de masse grasse et améliorer la silhouette.

### ETAT DE LA TECHNIQUE :

Définie comme la maladie au cours de laquelle un excédent de masse grasse s'est accumulé jusqu'à avoir des effets indésirables sur la santé, l'obésité est une priorité de l'Organisation Mondiale de la Santé (OMS), en terme de prévalence et de prise en charge. En effet, au niveau mondial, 300 millions d'individus sont obèses, avec une incidence dans les pays industrialisés d'environ 15 à 30 % de la population. On estime aussi que 43 millions d'enfants de moins de 5 ans présentent un surpoids et présentent ainsi un risque accru de développer une obésité à l'âge adulte. Par ailleurs la hausse de la prévalence de l'obésité dans la population générale s'accompagne d'un accroissement du nombre de mères présentant une surcharge pondérale ou obèses en début de grossesse, or les mères obèses sont bien plus susceptibles d'avoir des enfants obèses, surtout si elles souffrent de diabète gestationnel ou du syndrome métabolique avant leur grossesse. En France, le surpoids et l'obésité concernent ensembles plus de 46 % des adultes et 19% des enfants.

Le surpoids (ou surcharge pondérale) et l'obésité sont couramment définis par un Indice de Masse Corporelle (IMC), qui prend en compte le poids et la taille d'un sujet pour catégoriser sa corpulence. Exprimé en kg/m², l'IMC correspond au poids divisé par le carré de la taille. Selon les normes de l'OMS, pour un être humain adulte, un IMC compris entre 18,5 et 25 kg/m² correspond à une corpulence dite «normale», c'est à dire non associée à une augmentation de la charge de morbidité (effets indésirables sur la santé). Le surpoids est caractérisé par un IMC compris entre 25 et 30 kg/m², et l'obésité par un IMC supérieur à 30 kg/m².

Le tissu adipeux (ou masse grasse) est un tissu conjonctif contenant des cellules graisseuses, ou adipocytes, reliées par un fin tissu fibreux. Situé juste sous la peau, le tissu adipeux protège et isole la plus grande partie du corps, et sert également de réserve d'énergie. La masse grasse augmente par multiplication des adipocytes (hyperplasie adipocytaire) et/ou augmentation de la taille des adipocytes (hypertrophie adipocytaire). Chez l'adulte, l'augmentation de la masse adipeuse se fait essentiellement par hypertrophie adipocytaire, plus rarement par hyperplasie. Le pourcentage de masse grasse peut être estimé selon différentes techniques connues de l'homme du métier. On considère que chez l'homme, un pourcentage de masse grasse compris entre 15 et 20 % est dans la normalité, c'est-à-dire qu'il n'est pas associé à un état pathologique. Chez la femme les taux dans la normalité se situent entre 25 et 30 %. Au-delà de ces taux respectifs pour l'homme et la femme, l'excès de masse grasse est susceptible d'être associé à des états pathologiques sévères. L'impact de la surcharge de masse grasse (i.e. tissus adipeux) sur la santé dépend également de sa localisation corporelle. En effet, la graisse se répartit de différentes façons dans l'organisme. On définit ainsi un surpoids de type gynoïde, dans lequel la masse graisseuse s'accumule au niveau des hanches, des fesses et des jambes et un surpoids de type androïde, dans lequel l'excès de graisse se situe au niveau des organes abdominaux, sur la paroi abdominale et parfois sur le haut du dos. Des études épidémiologiques démontrent que le risque de développer des maladies métaboliques et/ou cardiovasculaires est particulièrement lié à la surcharge pondérale et à l'obésité de type androïde (adiposité abdominale). A ce titre, l'obésité abdominale (ou androïde) constitue le facteur central de la définition du syndrome métabolique et est donc associée à une morbidité importante.
Le surpoids et l'obésité sont donc également diagnostiqués par le tour de taille, ou le rapport tour de taille/tour de hanche, afin d'estimer directement l'adiposité abdominale et la présence d'un surpoids ou d'une obésité androïde. Chez la femme, le surpoids est caractérisé par un tour de taille compris entre 80 et 88 cm, et l'obésité par un tour de taille supérieur à 88 cm. Chez l'homme, le surpoids est caractérisé par un tour de taille compris entre 94 et 102 cm, l'obésité par un tour de taille supérieur à 102 cm. Considérant le rapport tour de taille/tour de hanche, l'obésité est définie selon l'OMS par un rapport supérieur à 0.9 chez l'homme et supérieur à 0.85 chez la femme. Chez l'enfant et l'adolescent, on utilise également l'IMC pour évaluer la corpulence. Dans l'enfance, l'IMC varie physiologiquement avec l'âge et le sexe. Les valeurs d'IMC doivent donc être reportées sur des courbes de percentiles d'évolution de la corpulence. En 2000, l'International Obesity Task Force (IOTF), sous l'égide de l'OMS, a proposé une définition internationale de l'obésité infantile (Cole et al., BMJ., 2000, 320(7244):1240-3). Les seuils de surpoids (obésité de degré 1) et d'obésité (obésité de degré 2) chez l'enfant sont définis respectivement par les courbes de percentiles rejoignant respectivement l'IMC de 25 et l'IMC de 30 à 18 ans. Ces courbes permettent d'assurer la continuité enfant-adulte. Le National Cholesterol Education Program Adult Treatment Panel III (NCEP-ATP III) retient également comme critère d'obésité chez l'enfant ou l'adolescent un tour de taille supérieur à 90 cm.

Dans la Région Européenne, deux grands facteurs de risque contribuent à un déséquilibre de la balance énergétique et au développement d'un surpoids voir d'une obésité ou à l'accroissement de la masse graisseuse: (i) un régime alimentaire à haute densité énergétique et apportant un faible sentiment de satiété, conséquence d'une proportion élevée d'énergie provenant de matières grasses (huiles végétales et graisse animales) (i.e., régime hyperlipidique), d'une importante consommation de sucre et d'une faible absorption de fibres, associé à une consommation accrue de boissons à haute teneur en sucre, éventuellement alcoolisée et (ii) une faible dépense énergétique liée à une sédentarité accrue. Ces deux facteurs échappent à la régulation biologique normale, rapide, de l'appétit et de l'absorption de nourriture, de sorte que les individus ont tendance à ne pas adapter leur consommation lorsque ces aliments et ces boissons sont proposés en permanence.

On peut également citer d'autres facteurs de risque, comme l'anxiété, le stress, ou la dépression, une susceptibilité familiale au surpoids ou à l'obésité (impliquant des marqueurs génétiques), un déséquilibre hormonal, la ménopause.

Les mesures hygiéno-diététiques, associant une alimentation équilibrée à la pratique régulière d'une activité physique, représentent l'approche la plus simple pour contrer le surpoids ou l'obésité. Or la pratique régulière d'un sport est contraignante et en outre, les bénéfices d'un régime restrictif sont souvent limités par un rebond du poids à la reprise d'une alimentation normale.

Il est donc important de développer des traitements complémentaires pour prévenir et traiter les troubles sévères du poids, et notamment la prise de masse grasse abdominale, dont l'impact en termes de morbidité et de mortalité est considérable.

Par ailleurs, la perte de poids, induite principalement par des régimes alimentaires restrictifs, est associée à une perte musculaire (masse maigre) importante, or pour des raisons médicales évidentes, la fonte musculaire doit être évitée car elle est toujours de mauvais pronostic.

L'accumulation d'amas graisseux (i.e. masse grasse ou encore tissu adipeux) localisés, comme par exemple, la cellulite, peut intervenir également de façon non pathologique (i.e. : non associée à une augmentation de la charge de morbidité) chez des personnes en bonne santé et de corpulence normale (selon les critères de l'OMS). De tels amas graisseux, bien que sans conséquence indésirable sur la santé, peuvent être considérés comme inesthétiques.
Chez la femme, par exemple, l'accumulation de la masse grasse intervient particulièrement aux niveaux de fesses et des hanches. Une telle accumulation est encore accentuée lors de la ménopause.

Il est donc également utile de développer des stratégies cosmétiques pour permettre aux individus en bonne santé, de conserver leur poids aussi bas que possible, dans la limite de valeurs non pathologiques, et/ou une silhouette mince dépourvue d'amas graisseux localisés, comme la cellulite, ou encore de perdre du poids superflu et/ou une masse grasse disgracieuse, comme la cellulite. Un tel traitement cosmétique pourra également s'adresser aux femmes lors de la ménopause, pour prévenir et/ou limiter la prise de masse grasse de type gynoïde ou androïde ainsi que chez la jeune femme souhaitant éliminer une accumulation inesthétique de masse grasse, par exemple après la grossesse.

De telles stratégies thérapeutiques ou cosmétiques seront d'autant plus utiles qu'elles permettront de cibler préférentiellement la perte de masse grasse tout en préservant la masse maigre.

### RESUME DE L'INVENTION :

La présente invention se rapporte à un produit de combinaison, comprenant à titre de substances actives, au moins :
(i) un inhibiteur de l'enzyme HMG-CoA-réductase, et
(ii) la citrulline ou l'un de ses sels;
pour son utilisation dans le traitement du surpoids, ou de l'obésité et/ou de la surcharge de masse grasse,
ledit produit ne comprenant pas d'arginine, et
la citrulline étant administrée à une dose quotidienne de 1 à 15 g/jour.
La présence de l'arginine dans un produit de combinaison selon l'invention est exclue.
Dans un mode de réalisation de l'invention, ledit produit de combinaison est caractérisé en ce que les substances actives consistent en :
i. un inhibiteur de l'enzyme HMG-CoA-réductase, et
ii. la citrulline.
Dans certains modes de réalisation, le produit de combinaison selon l'invention comprend au moins à titre de substances actives :
(i) l'atorvastatine ou une statine d'origine naturelle, ou sous forme d'extrait végétal, comme par exemple sous forme d'un extrait de levure rouge de riz et
(ii) la citrulline
Dans certains modes de réalisation de l'invention, le produit de combinaison selon l'invention peut se présenter sous la forme d'une composition unique (ou combinée) contenant au moins à titre de substances actives :
(i) ledit inhibiteur de l'enzyme HMG-CoA-réductase, et
(ii) ladite citrulline;
dans un milieu physiologiquement acceptable.
En particulier, le produit de combinaison selon l'invention peut être sous la forme d'une composition unique dont les substances actives (i) et (ii) consistent en :
(i) un inhibiteur de l'enzyme HMG-CoA-réductase, et
(ii) la citrulline et/ou un composé bioéquivalent de celle-ci;
dans un milieu physiologiquement acceptable.

Selon d'autres modes de réalisation, le produit de combinaison selon l'invention peut se présenter sous forme d'une trousse contenant:
(a) une composition incluant ledit inhibiteur de l'enzyme HMG-CoA-réductase dans un milieu physiologiquement acceptable ; et
(b) une composition incluant ladite la citrulline, dans un milieu physiologiquement acceptable ; lesdites compositions (a) et (b) étant chacune fournie sous une forme qui convient à une administration de façon séquentielle, séparée et/ou simultanée de l'une avec l'autre dans le temps.
Selon un mode de réalisation particulier, le produit de combinaison selon l'invention peut se présenter sous forme d'une trousse contenant:
(a) une composition consistant en un inhibiteur de l'enzyme HMG-CoA-réductase dans un milieu physiologiquement acceptable ; et
(b) une composition consistant en la citrulline, dans un milieu physiologiquement acceptable ;
lesdites compositions (a) et (b) étant chacune fournie sous une forme qui convient à une administration de façon séquentielle, séparée et/ou simultanée de l'une avec l'autre dans le temps.
Selon un mode de réalisation particulier de l'invention, le produit de combinaison est caractérisé en ce que les ingrédients actifs (i) et (ii) ou les compositions (a) et (b) sont dans un ou des milieux adaptés à l'administration par voie orale.
Lorsqu'il est utilisé pour le traitement thérapeutique de l'obésité ou du surpoids et de la surcharge de masse grasse, le produit de combinaison suivant la présente invention, est notamment destiné à des sujets stressés ou angoissés, et/ou ayant une alimentation riche en graisse ou en sucre, et/ou un mode de vie sédentaire, et/ou présentant des dérèglements hormonaux et/ou une susceptibilité familiale au surpoids ou à l'obésité, ou encore prenant des médicaments entraînant une prise de poids.
Dans certains modes de réalisation particulièrement préférés, le produit de combinaison selon l'invention est destiné à des sujets présentant des troubles du métabolisme du glucose, et notamment une hyperglycémie à jeun et/ou postprandiale élevée, étant entendu les taux de glycémie à jeun et/ou postprandiale sont inférieurs aux taux définissant un état diabétique.

La citrulline pourra être présente en tant qu'ingrédient (ii) dans un produit de combinaison de l'invention sous forme plus ou moins purifiée, notamment sous forme d'extrait végétal. Elle pourra également être sous forme libre ou sous forme de dérivés. Les dérivés de la citrulline acceptables selon l'invention incluent notamment les formes complexées, protégées (comme les formes estérifiées et les formes dans lesquelles la fonction amine est protégée par le radical tert-butoxycarbonyle, autrement dit Boc) ou les sels, ainsi que ses précurseurs, comme l'ornithine ou la glutamine.

La citrulline peut se présenter sous forme pure ou sous la forme d'un extrait végétal la contenant, en particulier d'un extrait de plante appartenant à la famille des cucurbitaceae. Typiquement, un produit selon l'invention est caractérisé en ce l'ingrédient actif (i) est administré à une dose quotidienne allant de 10 à 80 mg/jour, lorsqu'il s'agit de statine synthétique et l'ingrédient actif (ii) est administré à une dose quotidienne allant de 2 à 5 g/jour. L'inhibiteur de la HMG-CoA réductase peut notamment être choisi parmi le groupe constitué par les statines d'origine naturelle ou synthétique, les monacolines, la berbérine, et les policosanols et leurs mélanges en toutes proportions.
Un produit de combinaison particulièrement adapté à l'invention contient au moins l'atorvastatine en tant qu'inhibiteur de la HMG-CoA réductase.
Selon d'autres modes de réalisation avantageux de l'invention, l'inhibiteur de la HMG-CoA réductase est une statine naturelle, ou se présente sous la forme d'un extrait de levure rouge de riz, ou *monascus purpureus.*

La présente demande décrit également à un produit de combinaison comprenant au moins à titre de substances actives :
(i) un inhibiteur de la HMG-CoA réductase sous forme d'extrait végétal en contenant, de préférence un extrait de levure rouge de riz, et/ou un extrait végétal contenant des monacolines et/ou de la berbérine et/ou des policosanols, et
(ii) la citrulline de préférence sous forme d'extrait végétal en contenant ;
lesdits ingrédients (i) et (ii) étant dans une forme adaptée à une administration séquentielle, séparée et/ou simultanée de l'un avec l'autre.
Selon un mode de réalisation particulier dudit produit, la citrulline est sous forme pure. Dans un autre mode de réalisation, l'invention se rapporte à un produit de combinaison comprenant comme seuls ingrédients à titre de substances actives :
(i) un inhibiteur de la HMG-CoA réductase sous forme d'extrait végétal en contenant, de préférence un extrait de levure rouge de riz, et/ou un extrait végétal contenant des monacolines et/ou de la berbérine et/ou des policosanols, et
(ii) la citrulline, sous forme d'extrait végétal en contenant ou sous forme pure;
lesdits ingrédients (i) et (ii) étant dans une forme adaptée à une administration séquentielle, séparée et/ou simultanée de l'un avec l'autre.
Un tel produit pourra être sous forme d'une composition unique ou sous forme de compositions distinctes, tels que précédemment décrites.
Selon un mode de réalisation avantageux, les ingrédients (i) et (ii) sont dans un ou des milieux, suivant que le produit de combinaison est respectivement sous forme de composition unique ou de compositions distinctes, adaptées à l'administration par voie orale.
A titre d'exemple, dans un tel produit de combinaison, la composition (a) peut se présenter sous forme unitaire de capsule et la composition (b) peut se présenter sous forme unitaire de sachet. De façon préférée, un tel produit de combinaison est caractérisé en ce l'ingrédient actif (i) est un extrait standardisé contenant 2 % de monacoline K.

La présente invention concerne également un procédé de traitement cosmétique, pour améliorer l'apparence d'un individu, par exemple pour affiner la silhouette et/ou diminuer ou limiter les amas graisseux localisés ou lipodystrophie, et/ou stimuler la perte de poids superflu et/ou de cellulite, et/ou limiter son accumulation, chez les individus en bonne santé, comprenant l'administration d'une association comprenant au moins à titre de substance active :
(i) un inhibiteur de la HMG-CoA réductase sous forme d'extrait végétal en contenant, de préférence un extrait de levure rouge de riz, et/ou un extrait végétal contenant des monacolines et/ou de la berbérine et/ou des policosanols
(ii) la citrulline, de préférence sous forme d'extrait végétal en contenant ;
et le renouvellement de ladite administration jusqu'à obtention de l'effet cosmétique désiré. De préférence, ladite association se présente sous forme d'un produit de combinaison dans lequel lesdits ingrédients (i) et (ii) sont dans une forme adaptée à une administration par voie orale, séquentielle, séparée et/ou simultanée de l'un avec l'autre dans le temps.
De façon préférée, dans un tel procédé de traitement cosmétique l'ingrédient actif (i) est administré à une dose comprise entre 100 et 600 mg de une à trois fois par jour et l'ingrédient actif (ii) est administré à une dose quotidienne, comprise entre 2 et 5 g/jour.
Un tel traitement cosmétique est destiné aux individus dont le poids et la surcharge graisseuse ne sont pas associés à une augmentation de la charge de morbidité et en particulier aux individus ne présentant pas de troubles du métabolisme du glucose, une résistance à l'insuline, un syndrome métabolique, un diabète ou des troubles vasculaires.

L'invention concerne enfin l'utilisation d'une association comprenant au moins (i) un inhibiteur de l'HMG CoA réductase, et (ii) la citrulline,
pour améliorer l'apparence d'un individu, par exemple pour affiner la silhouette et/ou diminuer ou limiter les amas graisseux localisés, ou lipodystrophie, et/ou stimuler la perte de poids superflu et/ou de cellulite et/ou limiter son accumulation, chez les individus en bonne santé. De façon préférée, l'association se présentera sous la forme d'un produit de combinaison tel que précédemment défini. De façon préférée encore, le produit de combinaison sera adapté à la prise par voie orale.

Les différents modes de réalisations de l'invention sont particulièrement décrits dans la description détaillée ci-après. Ces modes de réalisation peuvent être pris séparément ou en combinaison les uns avec les autres.

### DESCRIPTION DETAILLEE DE L'INVENTION :

Dans la présente invention, la demanderesse a mis en évidence que l'association d'au moins (i) un inhibiteur de la HMG-CoA-réductase et (ii) de la citrulline, présentait un effet synergique sur la perte de poids et la réduction de la masse grasse, tout en préservant la masse maigre.

Par effet synergique, il est entendu selon la présente invention que l'association d'au moins un inhibiteur de la HMG-CoA-réductase et de la citrulline présente un effet sur la diminution du poids ou de la masse grasse, au cours du traitement qui est supérieur aux effets cumulés d'un traitement (a) comprenant l'administration d'un inhibiteur de la HMG-CoA-réductase, non associé à la citrulline, et inversement d'un traitement (b) comprenant l'administration de la citrulline, non associé à un inhibiteur de la HMG-CoA-réductase.

La citrulline est un intermédiaire du cycle de l'urée dans le foie, au cours duquel l'ammoniac en excès est éliminé. Précurseur de l'arginine elle serait également impliquée dans la synthèse du monoxyde d'azote (Curis E. et al., Amino Acids, 2005 Nov, 29(3): 177-205). Certaines études suggèrent que la citrulline pourrait avoir un effet sur la masse maigre. En effet, la citrulline, pourrait stimuler la synthèse protéique et modulerait notamment le métabolisme protéique musculaire (Osowska S. et al., Citrulline modulates muscle protein metabolism in old malnourrished rats, Am. J. PhysioL Endocrinol. Metab., 2006 April). Une revue récente évoque un rôle régulateur du monoxyde d'azote sur le métabolisme énergétique et suggère que l'arginine ou son précurseur, la citrulline pourraient jouer un rôle chez les mammifères (animaux et/ou humains) en prévention, ou pour le traitement, du syndrome métabolique chez les individus obèses et contribuer à réduire la proportion de masse grasse chez les animaux d'élevage (Jobgen W.S. et al., J. Nutr. Biochem., 2006 ; 17(9) : 571-88).
Les inhibiteurs de l'enzyme HMG-CoA-réductase, et en particulier les statines, sont particulièrement prescrits en tant qu'hypolipémiants dans le traitement des dyslipidémies, en particulier l'hypercholestérolémie, en prévention primaire et secondaire des accidents cardiovasculaires. En revanche, un effet des inhibiteurs de l'enzyme HMG-CoA réductase sur la masse grasse n'a, à la connaissance de la demanderesse jamais été démontré. Au contraire, une étude de Bucek et al., (Vasa, J. for Vascular Disease, german, 2006, 35(2) :92-5) rapporte les résultats d'études cliniques sur l'influence d'inhibiteurs de l'enzyme HMG-CoA-réductase sur la masse grasse corporelle, qui concluent que la prise de statine, en particulier de l'atorvastatine et de la simvastatine à long terme n'a aucune influence positive sur la masse grasse corporelle.
CN 101731699 décrit des compositions comprenant de nombreux ingrédients, comme les jus de pomme, carotte, citron et la levure rouge de riz. De nombreuses indications thérapeutiques et cosmétiques sont proposées et notamment dans un but anorexigène, ce qui entrainerait nécessairement une perte de masse maigre.
CN102113648 décrit également des compositions à visée notamment anorectique, comprenant de nombreux ingrédients, comme de la poudre de riz noir, des fibres, du soja, de la poudre de haricot, de la poudre de citrouille, de la taurine.
US2005/0288737 propose d'associer l'arginine avec un inhibiteur de l'HMG CoA réductase pour le traitement du surpoids, mais exclut l'association de la seule citrulline avec un inhibiteur de l'HMG CoA réductase.
La mise en évidence selon l'invention, que l'association d'au moins (i) un inhibiteur de la HMG-CoA-réductase et (ii) de la citrulline présente un effet synergique sur la perte de poids et la réduction de la masse grasse sans perte de la masse maigre est donc tout à fait surprenante.
Une telle association sera donc destinée au traitement (préventif ou curatif) du surpoids ou de l'obésité et/ou de la surcharge de masse grasse. En particulier, un produit de combinaison tel que défini dans la présente demande et associant (i) un inhibiteur de l'HMG CoA réductase et (ii) la citrulline sera utilisée pour limiter la prise de masse grasse ou réduire la masse grasse installée sans altérer la masse maigre, dans le traitement du surpoids et/ou de l'obésité.

Un tel produit de combinaison pourra également être utilisé dans le cadre d'un procédé (ou méthode) de traitement cosmétique chez des sujets en bonne santé, c'est-à-dire dont le poids et/ou la surcharge de masse grasse ne sont pas associés à une augmentation de la charge de morbidité. De préférence, on entend par sujet en bonne santé selon l'invention, des individus qui ont une corpulence considérée comme normale selon les critères de l'OMS. Dans le cadre d'une utilisation cosmétique, l'association selon l'invention se présentera de préférence sous une forme adaptée à l'administration par voie orale, comme par exemple un complément alimentaire ou un aliment fonctionnel.
Dans la suite de la présente demande, le terme « produit de combinaison » est également utilisé en tant qu'équivalent de « l'association » telle que défini ci-dessus.

Par citrulline, il est entendu selon l'invention les formes énantiomères D, L ainsi que leurs mélanges en toutes proportions. Dans un produit de combinaison selon l'invention, la citrulline peut être sous forme pure et/ou contenue dans un extrait végétal. Elle pourra être sous forme libre et/ou sous forme de l'un au moins de ses dérivés. Lesdits dérivés consistent en des précurseurs de la citrulline, comme l'ornithine ou la glutamine et/ou sont des formes modifiées de la citrulline (ou de ses précurseurs), par exemple pour améliorer sa stabilité. A titre d'exemple de telles modifications, on citera les sels de citrulline (ou de ses précurseurs), les formes complexées et ses formes dans lesquelles le groupement carboxyle a été estérifié ou transformé en groupement amide et/ou dont un groupement amine ou amide a été substitué, notamment protégé, par exemple alkylé, de préférence méthylé, arylé, ou acylé, de préférence acétylé.
Selon l'invention, les groupements amine, et carboxyle de la citrulline (ou de ses précurseurs) peuvent donc être présents sous forme de sel. A titre d'exemple, on citera les sels d'acides inorganiques ou organiques, comme l'acide oxalique, l'acide lactique, l'acide acétique, l'acide malique, l'acide citrique, l'acide fumarique, l'acide phosphorique, HCl, HBr, l'acide p-toluène sulfonique. Sont également appropriés les sels des bases telles que la soude, la potasse, l'hydroxyde de calcium, l'hydroxyde d'ammonium, les malates, ou les sels d'amines, comme à titre d'exemple, l'éthanolamine, la di-éthanolamine, la tri-éthanolamine, la N-méthylglucamine, la tri-(hydroxyméthyl)-méthylamine ou la bis-cyclohexamine.
La citrulline ou ses dérivés, tels que définis ci-dessus par exemple, peuvent être disponibles commercialement ou peuvent être synthétisés selon des méthodes connues de l'homme du métier. Les sels peuvent être achetés ou bien peuvent être obtenus aisément par mélange des acides aminés avec un autre acide ou une base dans des solvants tels que l'éthanol, ou le dioxane ou le dialkyléther ou le tétrahydrofurane.
La citrulline selon l'invention pourra également être issu de produits naturels comme les plantes (incluant les fruits). En particulier, elle pourra être extraite de plantes appartenant à la famille des *cucurbitaceae,* comme notamment le concombre, la gourde, le melon brodé, le melon amer et la pastèque. Suivant les différents modes de réalisation de l'invention, la citrulline pourra donc être sous forme pure ou sous forme d'extrait végétal en contenant, notamment des extraits de plantes appartenant à la famille des *cucurbitaceae,* comme le concombre, la gourde et de préférence le melon (brodé ou amer) et la pastèque. Par citrulline sous forme pure, il est entendu selon l'invention la citrulline sous forme libre et/ou au moins l'un quelconque de ses sels, ou dérivés, tels que définis précédemment par exemple.

Par inhibiteur de l'HMG-CoA réductase on entend selon l'invention, un composé capable de ralentir ou de s'opposer à la production d'acide mévalonique à partir de l'HMG co-Enzyme A en, une étape déterminante de la biosynthèse du cholestérol. Un tel composé pourra agir en (i) inhibant de l'expression génique de la HMG-CoA réductase, ou (ii) en ralentissant ou en s'opposant à la réaction enzymatique catalysée par L'HMG-CoA réductase, c'est-à-dire, la conversion de l'HMG co-Enzyme A en acide mévalonique. Typiquement un inhibiteur selon l'invention pourra empêcher la fixation du substrat sur le site actif de l'enzyme en se fixant à sa place (inhibition compétitive), ou provoquer une déformation de l'enzyme rendant celle-ci inactive (inhibition allostérique, incompétitive ou non compétitive). De tels composés pourront se présenter sous la forme de molécules pures ou de mélanges plus ou moins complexes (contenant au moins un inhibiteur de l'HMG Co-A réductase) et notamment sous la forme d'extraits végétaux.
Parmi les inhibiteurs compétitifs de l'HMG-CoA réductase convenant à l'invention on peut citer des composés appartenant à la classe des statines. Les statines ont en commun une structure chimique constituée par un cycle lactone ouvert ou fermé, lié notamment à un groupement de nature plus ou moins hydrophobe. Selon certains modes de réalisation, les statines peuvent être choisies par exemple, parmi le groupe des statines de synthèse, comprenant l'atorvastatine, la pravastatine, la fluvastatine, la rosuvastatine, la lovastatine et la simvastatine et leurs mélanges en toutes proportions. Cette liste n'est pas limitative et toutes les autres statines connues de l'homme du métier présentant un effet inhibiteur de l'enzyme HMG-CoA réductase peuvent également être utilisées selon l'invention. Selon un mode de réalisation, la statine est l'atorvastatine. D'autres statines convenant à l'invention sont également les statines d'origines naturelles, ainsi que leurs mélanges en toutes proportions. On citera par exemple les monacolines. Les monacolines, et notamment la monacoline K (ou lovastatine) peuvent être issues de levure rouge de riz, obtenue par fermentation de la levure *Monascus Purpureus,* sur du riz. De telles statines pourront par exemple être utilisées dans certains modes de réalisation de l'invention sous forme pure ou sous forme d'un extrait de levure rouge de riz en contenant (typiquement du riz fermenté avec la levure *Monascus Purpureus,* séché et réduit en poudre). De préférence un extrait de levure rouge de riz selon l'invention est un extrait standardisé contenant entre 1 et 4 % de monacoline K, plus particulièrement entre 1 et 3 % de monacoline K et typiquement contenant 2 % de monacoline K.

D'autres inhibiteurs de l'enzyme HMG-CoA-réductase convenant à l'invention sont par exemple le policosanol (ou polycosanol) et la berbérine. Le policosanol est un mélange d'alcools primaires à longue chaîne (comme l'octacosanol et le triacosanol), de formule : CH₃-(CH₂)ₙ-CH₂OH
(n étant compris entre 24 et 34), obtenu à partir de cire de plantes comme la canne à sucre et l'igname, ou encore de cire d'abeille. La berbérine est un alcaloïde issu de plantes de la famille des *berbéridacées.* Le policosanol comme la berbérine pourront, dans certains modes de réalisation, être utilisés sous forme pure ou sous forme d'extraits végétaux en contenant.

Selon l'invention, un extrait végétal est obtenu par extraction solide/liquide, éventuellement suivie d'étapes de purification. L'extraction solide-liquide se définit comme une opération de séparation de constituants contenus dans un corps solide par solubilisation avec un solvant par exemple aqueux ou alcoolique. Par extrait végétal il est également compris selon l'invention les plantes (en totalité ou en parties) séchées ou déshydratées et broyée ou réduite en poudre. De tels extraits peuvent être obtenus par pressage, avec ou sans extraction par solvant, expression, hydrodistillation, extraction aqueuse ou alcoolique, filtration fermentation, macération. Cette liste n'étant en aucun cas limitative. Les extraits selon l'invention peuvent être sous forme de jus, concentré aromatique, huile, tisane, infusion, macérât, extrait sec, mou, extrait hydro alcoolique, ou de lyophilisat. De même cette liste n'est également en aucun cas limitative.

Les inhibiteurs de l'enzyme HMG-CoA réductase et leurs mélanges en toutes proportions, pourront être utilisés sous forme de leurs sels comme par exemple, et sans s'y limiter, leurs sels d'acides inorganiques ou organiques, comme l'acide oxalique, l'acide lactique, l'acide acétique, l'acide citrique, l'acide malique, l'acide fumarique, l'acide phosphonique, HCl, HBr, l'acide p-toluène sulfonique. Sont également appropriés les sels des bases telles que le sodium, le potassium, l'hydroxyde de calcium, l'ammonium, le malate, ou les sels d'amines, comme à titre d'exemple, l'éthanolamine, la di-éthanolamine, la tri-éthanolamine, la N-méthylglucamine, la tri-(hydroxyméthyl)-méthylamine ou la bis-cyclohexamine.
De façon générale selon l'invention, la citrulline ainsi que les inhibiteurs de l'enzyme HMG-CoA réductase pourront être utilisés sous forme de sels physiologiquement acceptables. Par sels physiologiquement acceptable on entend des composés qui possèdent l'activité pharmacologique ou cosmétique souhaitée du composé parent, sans présenter d'activité toxique lors de leur administration.

L'invention concerne un produit de combinaison comprenant à titre de substances actives, au moins :
(i) un inhibiteur de l'enzyme HMG-CoA réductase, et
(ii) la citrulline,
pour utilisation dans le traitement du surpoids ou de l'obésité et/ou le traitement de la surcharge de masse grasse, ledit produit ne comprenant pas d'arginine et la citrulline étant administrée à une dose quotidienne de 1 à 15 g/jour. Selon un mode de réalisation particulier, le produit de combinaison est utilisé pour réduire la masse grasse ou prévenir son apparition sans diminution de la masse maigre. Un tel produit de combinaison sera donc préférentiellement adapté à des sujets obèses ou en surpoids.

Il est également décrit ici l'utilisation d'un produit de combinaison comprenant à titre de substances actives, au moins :
(i) un inhibiteur de l'enzyme HMG-CoA réductase, et
la citrulline, pour l'obtention d'un médicament destiné au traitement du surpoids ou de l'obésité et/ou le traitement de la surcharge de masse grasse.

L'invention est particulièrement destinée à l'homme mais pourra également être destinée à tous les mammifères, en particulier les animaux de compagnie, d'élevage ou de course.

Selon les besoins de l'invention, le terme traitement inclut à la fois les traitements préventifs et curatifs. Ce terme inclut également le traitement des patients à risques de développer une surcharge de masse grasse, et/ou un surpoids ou une obésité.

Par « traitement du surpoids ou de l'obésité », il est entendu que l'administration d'un produit de combinaison, tel que présentement défini, et selon une posologie déterminée par l'homme du métier, permet de diminuer le poids des sujets (mammifère : être humain ou animal) traités. Un tel produit permet également de prévenir la prise de poids et/ou de conserver un poids stable, ou aussi bas que possible, et/ou de retarder le développement d'un surpoids ou d'une obésité, notamment chez des sujets à risques de développer un surpoids ou une obésité.

Par traitement de la surcharge de masse grasse, il est entendu que l'administration d'un produit de combinaison tel que présentement défini, et selon une posologie déterminée par l'homme du métier, permet de diminuer la masse grasse des sujets (être humain ou animal) traités. Un tel produit permet également de prévenir la prise de masse grasse et/ou de conserver une masse grasse aussi bas que possible, ou de retarder l'accroissement de la masse grasse, notamment chez des sujets présentant des facteurs de risques de développer un surpoids ou une obésité et/ou de développer une surcharge de masse grasse de nature pathologique (i.e. : associée à une augmentation de la charge de morbidité), comme souvent dans le cas de l'adiposité abdominale.
Préférentiellement la perte de masse grasse intervient sans perte de masse maigre (sans fonte musculaire), la perte de poids est donc associée majoritairement à une perte spécifique de masse grasse.

Des sujets à risques de développer un surpoids, ou une obésité, ou présentant des facteurs de risque de développer un surpoids ou une obésité sont par exemple des sujets présentant un déséquilibre de la balance énergétique (i.e. : le rapport entre l'apport et la dépense énergétique) lié à une alimentation hypercalorique (i.e. : riche en graisse et/ou en sucre) et/ou à un mode de vie sédentaire, des sujets anxieux, dépressifs, stressés, présentant un déséquilibre hormonal, ou une susceptibilité familiale (génétique) à l'obésité, ou prenant certains médicaments induisant une prise de poids comme les glucocorticoïdes ou les antidépresseurs.

L'association d'au moins un inhibiteur de la HMG-CoA-réductase et de la citrulline est également particulièrement destinée aux individus présentant des troubles du métabolisme du glucose. En effet, la demanderesse a démontré dans la présente demande qu'une telle association présente également un effet synergique sur l'amélioration de la tolérance au glucose. Un tel effet synergique est tout à fait surprenant, car à la connaissance de la demanderesse, un effet de la citrulline sur les troubles du métabolisme du glucose n'a jamais été rapporté. En outre, les données de la littérature relatives à l'effet des statines, et en particulier de l'atorvastatine sur les troubles du métabolisme autres que les dyslipidémies sont très controversées. Des études très récentes démontrent en effet des effets métaboliques antagonistes de différents types de statines et questionnent leur utilisation à grande échelle. Les revues de Kok et al. (Atherosclerosis, 2011, 215(1):1-8) et Kopstapanos et al. (Curr Vasc Pharmacol, 2011, 215(1) :1-8) rapportent notamment les conclusions de plusieurs études cliniques et soulignent que certaines statines n'ont aucun effet sur la sensibilité à l'insuline, voir même augmenteraient la résistance à l'insuline et notamment, que l'atorvastatine, la rosuvastatine et la simvastatine augmenteraient la survenue du diabète de type 2. Des résultats expérimentaux suggèrent en outre, que le caractère lipophile de certaines statines, ainsi que l'inhibition de la HMG-CoA-réductase, seraient des facteurs responsables d'un impact négatif des statines sur le métabolisme du glucose (Kopstapanos et al., Curr Vasc Pharmacol, 2011, 215(1) :1-8).
Un produit de combinaison comprenant les ingrédients (i) et (ii) sous l'une des formes définies dans la présente demande pourra donc être utilisé pour le traitement et/ou la prévention des troubles du métabolisme du glucose, en particulier pour le traitement de l'intolérance au glucose, ou de la résistance à l'insuline.
Comme il a été montré que la combinaison de l'invention préservait la masse maigre, l'utilisation d'une telle combinaison sera particulièrement indiquée pour des sujets âgés présentant une intolérance ou glucose et/ou une résistance à l'insuline. En effet, de tels patients sont particulièrement à risque de présenter ou de développer une perte de masse maigre (masse musculaire) et/ou une dénutrition protéique associée aux régimes restrictifs (qui sont d'ailleurs interdits chez la personne âgée selon les recommandations de la HAS (Raynaud-Simon et al., 2007).
Par sujets âgés, on entend selon l'invention, des sujets de 65 ans ou plus, de préférence des sujets de 70 ans ou plus et notamment les sujets ayant au moins 75 ans.
Selon la présente invention, les troubles du métabolisme du glucose (Impaired Glucose Metabolism, IGM) sont définis comme des taux de glucose dans le sang supérieurs à la normal mais inférieurs à ceux requis pour l'établissement d'un diagnostic de diabète de type II, ou diabète « sucré ». La prévalence des troubles du métabolisme du glucose varie selon les différents pays mais est en général 2 à 3 fois plus importante que celle du diabète. Jusqu'à récemment, les sujets présentant des troubles du métabolisme du glucose étaient considérés comme prédiabétiques, mais les résultats d'études épidémiologiques démontrent que ces sujets sont en réalités hétérogènes quant aux risques de développer un diabète et/ou des pathologies cardiovasculaires.
Parmi les sujets présentant des troubles du métabolisme du glucose, 58 % environ présentent une intolérance au glucose (Impaired Glucose Tolerance, IGT), 29 % présentent une hyperglycémie modérée à jeun (Impaired Fasting glycemia, IFG) et 13 % présentent les deux troubles (IGT/IFG). L'association selon l'invention est donc destinée aux individus présentant l'un et/ou l'autre de ces troubles du métabolisme du glucose.
L'IGT est caractérisée par une hyperglycémie post-prandiale élevée. La mesure l'IGT est standardisée par le test oral de tolérance au glucose (Oral Glucose Tolerance Test, OGTT), mesuré deux heures après la prise d'une dose de glucose (1.75 g de glucose par kg, généralement 75 g chez l'homme adulte). Chez des sujets sains, le taux est habituellement inférieur à 7.8 mmol/l (140 mg/dl). Des taux allant de 7.8 à 11.1 mmol/l (140 à 200 mg/dl) traduisent une intolérance au glucose. Des taux de glucose au-delà de ce dernier seuil confirment un diagnostic de diabète. Chez le sujet en bonne santé, la glycémie à jeun est ordinairement inférieure à 6.1 mmol/l (110 mg/dl). L'IFG est définie par des taux de glucose sanguin à jeun allant de 6.1 à 7 mmol/l (110 à 125 mg/dl). Au-delà de 7.0 mm/l (126 mg/dl), la glycémie à jeun traduit un état diabétique.
Selon l'invention, et à moins qu'il n'en soit spécifié autrement, les termes « compris entre » et « allant de (...) à » doivent être compris comme intégrant les bornes de l'intervalle.

Un produit de combinaison pour utilisation selon l'invention est susceptible de prendre plusieurs formes. De façon générale, il est prévu selon l'invention :
(1) une composition comprenant au moins en tant qu'ingrédients actifs (i) un inhibiteur de la HMG-CoA réductase, et (ii) la citrulline, dans un milieu physiologiquement acceptable.
   Une telle composition est également appelée composition (ou préparation) unique (ou combinée).
(2) Une trousse comprenant en tant qu'ingrédients actifs:
   (i) au moins un inhibiteur de l'HMG-CoA réductase, éventuellement dans un milieu physiologiquement acceptable ; et
   (ii) au moins la citrulline, éventuellement dans un milieu physiologiquement acceptable;
   lesdits ingrédients actifs (i) et (ii) étant chacun fourni sous une forme qui convient à une administration conjointe de l'un avec l'autre.

L'expression trousse est utilisée ici en tant que synonyme du terme coffret ou kit et correspond à un ensemble de produits présentés conjointement.
De façon préférée, les ingrédients actifs (i) et (ii) peuvent être respectivement sous forme de compositions (a) et (b) les contenant au moins, et dans lesquelles lesdits ingrédients actifs (i) et (ii) sont dans un milieu physiologiquement acceptable.

Selon un autre aspect de l'invention, il est prévu une méthode de fabrication d'une trousse, telle que défini précédemment, ladite méthode comprenant une étape de conditionnement de l'ingrédient actif (i) et de l'ingrédient actif (ii), rendant ainsi les deux l'ingrédient actifs adaptés à une administration conjointe de l'un avec l'autre.
Les compositions (a) et (b) telles que précédemment définies peuvent être:
(i) sous forme de compositions séparées (i.e. : indépendantes l'une de l'autre), ultérieurement associées pour une utilisation en association l'une avec l'autre, dans le cadre d'un traitement de combinaison.
(ii) présentées ensemble dans un même contenant sous forme de compositions séparées dans un conditionnement combiné pour une utilisation en association l'une avec l'autre, dans le cadre d'un traitement de combinaison.

Une trousse selon l'invention pourra également contenir des instructions pour l'utilisation des composants qu'elle contient.

Dans le cadre des produits de combinaison sous forme de trousse, l'expression « administration conjointe » selon la présente invention inclut que les ingrédients actifs (i) et (ii) ou les compositions (a) et (b) comprenant respectivement au moins (i) un inhibiteur de la HMG-CoA réductase, en mélange avec un milieu physiologiquement acceptable et (ii) la citrulline, en mélange avec un milieu physiologiquement acceptable, sont administrés séquentiellement (i.e. : successivement, de façon étalée dans le temps), séparément et/ou simultanément pendant la période de traitement.
Conformément au produit de combinaison selon l'invention, le terme « administration conjointe », inclut que les ingrédients actifs (i) et (ii) du produit de combinaison sont administrés (éventuellement de façon répétée) soit ensembles, soit séparément, de façon suffisamment rapprochée dans le temps, pour permettre un effet bénéfique synergique entre les deux composants, tel que défini précédemment. La détermination d'un tel effet synergique dépendra notamment de l'effet recherché, et/ou du patient, et/ou de la formulation des composants.
Les compositions selon l'invention peuvent ainsi être administrées de façon simultanées, sous forme d'une composition unique ou bien sous forme de compositions distinctes. Lorsque les compositions sont distinctes elles peuvent également être administrées de façon séquentielle, par exemple à des moments différents de la journée, (ainsi une composition comprenant au moins l'un des ingrédients actifs (i) ou (ii) pourrait être administrée le matin et l'autre pourrait être administrée le midi) et/ou à des fréquences quotidiennes.
L'expression « en association l'une avec l'autre » inclut que l'une ou l'autre des deux compositions (a) ou (b) peut être administrée (éventuellement de façon répétée voire continue) avant, après, et/ou en même temps que l'administration de l'autre composition. Il est par exemple envisageable que chacune des compositions (a) et (b) soient administrées à des moments différents de la journée, ou que l'une des deux compositions (a) ou (b) soient prise à une fréquence différente de l'autre. A titre d'exemple, une composition (a) comprenant au moins un inhibiteur de la HMG-CoA réductase pourrait être administrée quotidiennement, à raison d'une fois par jour, et une composition (b) comprenant au moins la citrulline pourrait être prise quotidiennement à raison de 1 à 3 fois par jour.

La formulation des produits de combinaison selon l'invention ainsi que la nature et la quantité des ingrédients actifs dans la composition unique ou les compositions de la trousse selon l'invention dépendent de la finalité thérapeutique ou cosmétique du traitement.

Les ingrédients actifs (i) et (ii) des compositions respectives (a) et (b) peuvent être dans des milieux physiologiquement acceptables de nature similaire ou différente. Un milieu physiologiquement acceptable est un milieu n'ayant aucun effet toxique ou préjudiciable dans les conditions d'utilisation et inerte vis-à-vis des ingrédients actifs du produit de combinaison. Un tel milieu peut par exemple comprendre différents adjuvants selon sa finalité thérapeutique ou cosmétique, comme de façon non limitative, des arômes, des colorants, des charges, des conservateurs, des diluants, des agents mouillants ou de suspension, etc. De tels milieux peuvent également permettre une libération immédiate, modifiée ou retardée de l'un ou l'autre des ingrédients actifs, que ceux-ci soient présentés ensemble sous forme de composition unique, ou séparément sous forme de trousse.

Dans un mode de réalisation précité, l'invention concerne un produit de combinaison comprenant à titre de substances actives, au moins :
i. un inhibiteur de l'enzyme HMG-CoA-réductase, et
ii. la citrulline ou l'un de ses sels,
pour utilisation dans le traitement du surpoids, de l'obésité ou de la surcharge de masse grasse,
ledit produit ne comprenant pas d'arginine, et
la citrulline étant administrée à une dose quotidienne de 1 à 15 g/jour.

Dans ce mode de réalisation de l'invention pour utilisation dans le traitement du surpoids, de l'obésité ou de la surcharge de masse grasse, le produit de combinaison selon l'invention peut comprendre, outre les ingrédients actifs (i) et (ii), respectivement l'inhibiteur de l'HMG-CoA réductase et la citrulline, d'autres ingrédients actifs, ou composés, supplémentaires sous réserve que lesdits ingrédients actifs, ou composés, supplémentaires ne nuisent pas à l'activité thérapeutique ou cosmétique de l'association selon l'invention. Ces dits ingrédients actifs supplémentaires peuvent être ajoutés aux ingrédients (i) et (ii) dans le cas de la composition unique ou à l'une et/ou l'autre des compositions (a) et (b) dans le cas de modes de réalisation sous forme de trousse.
Avantageusement, un ingrédient actif supplémentaire stimule la perte de poids ou de masse grasse et/ou aide à la prévention de la prise de poids ou de masse grasse et/ou aide à l'amélioration de la tolérance au glucose.
Dans le cas des formes pharmaceutiques (i.e. : à finalité thérapeutique) de l'invention, d'autres ingrédients actifs peuvent également être des hypolipémiants comme les inhibiteurs de l'absorption intestinale du cholestérol ou les fibrates, des médicaments améliorant la sensibilité à l'insuline ou stimulant la production d'insuline, comme les médicaments de la famille des biguanides, des glitazones, des hypoglycémiants comme les sulfamides. Tout composé supplémentaire présentant un intérêt nutritionnel et/ou stimulant la perte de poids ou de masse grasse ou présentant des propriétés bénéfiques pour le métabolisme du cholestérol, des lipides et/ou du glucose peut également être ajouté aux produits de combinaison de l'invention sous forme pharmaceutique ou cosmétique. Le produit de combinaison pour utilisation selon l'invention pourra comprendre, à titre d'exemple, des vitamines, des sels minéraux, des acides aminés essentiels, des acides gras essentiels, des oligoéléments, divers extraits végétaux, des fibres, des antioxydants, des flavonoïdes. Un produit de combinaison pour utilisation selon l'invention pourra comprendre en particulier des composants naturels aux propriétés hypolipémiantes, comme la gomme guggul ou les extraits de figuier de barbarie (nopal), anorexigène, comme des extraits d'hoodia, d'eucalyptus, de laurier sauce, de coca, de catharanthus, de phyllantus niruri, d'orthosiphon, d'algues, de caroube, de konjac, brûle-graisse (ou augmentant la thermogénèse), comme des extraits de fucus, d'algues marines, de thé vert, de maté, de guarana, de coleus, de garcinia de caféine, dépuratives, comme des extraits de pissenlit, artichaut, romarin, chardon marie, curcuma, hercampuri (gentianella) ou calmantes pour diminuer le stress souvent en cause dans l'obésité et permettre de mieux supporter le régime hypocalorique, comme des extraits de millepertuis (Hypericum), pavot de Californie (Eschscholtzia), valériane (Valeriana), tilleul (Tilia).

Des formulations pharmaceutiques ou cosmétiques adaptées peuvent être disponibles dans le commerce, ou être décrite dans la littérature, comme par exemple pour les formulations pharmaceutiques, dans Remington - The Science and Practice of Pharmacy, 19th ed., MAck Printing Company, Easton, Pennsylvania (1995) et dans Martindale - The Complete Drug Reference (34ème édition) ainsi que les documents qu'ils référencent. Enfin, la préparation de formulations adaptées, et en particulier d'une composition unique comprenant les ingrédients actifs (i) et (ii) peut être réalisée par l'homme du métier selon des techniques de routine.
Il est entendu que les différents ingrédients actifs d'une trousse selon l'invention peuvent se présenter dans des formes pharmaceutiques ou cosmétiques identiques ou distinctes et peuvent être administrés selon des voies identiques ou différentes.

La composition unique ou les compositions séparées d'un kit selon l'invention sont administrées préférentiellement de façon locale ou systémique, par exemple par voie orale, entérale, sublinguale, intraveineuse ou intra-artérielle, intra-musculaire, cutanée, sous-cutanée, transmuqueuse, rectale, transdermale, nasale, pulmonaire, topique ainsi que par toute voie parentérale, sous une forme posologique physiologiquement acceptable. Les modes d'administration préférés sont les voies entérale, en particulier orale, et intraveineuse. A titre d'exemple, une composition (a) pourra être administrée par voie intraveineuse en continue ou par bolus, et une composition (b) pourra être administrée quotidiennement par voie orale.
Pour l'administration entérale, orale, sublinguale, parentérale, sous-cutanée, intramusculaire, intraveineuse, ou intra-artérielle, nasale, pulmonaire, topique, transdermique, locale ou rectale, les compositions de l'invention peuvent être administrées sous forme unitaire en mélange avec des milieux connus de l'homme du métier, physiologiquement acceptables, aux êtres humains ou aux animaux.

De façon préférée, un produit de combinaison selon l'invention est administré par voie orale. Les formes appropriées pour la voie orale sont par exemple les comprimés, les gélules, les pastilles, les poudres, les granulés, les lyophilisats, les solutés buvables et les sirops. Les comprimés, poudres, les granulés, les lyophilisats, les solutés buvables et les sirops constituent la forme pharmaceutique ou cosmétique adaptée à la voie orale actuellement préférée. Dans le cas de la préparation d'une composition solide sous forme de comprimés, on pourra par exemple mélanger l'ingrédient actif principal (i) ou (ii) avec un véhicule physiologiquement acceptable tel que la gélatine, l'amidon, le lactose, le stéarate de magnésium, le talc, la gomme arabique ou analogies. Les comprimés peuvent être de nature variée, à libération immédiate, contrôlée ou retardée et éventuellement sous forme effervescente ou orodispersible. On obtient une préparation en gélule en mélangeant l'ingrédient actif (i) ou (ii) avec un diluant et en versant le mélange obtenue dans des gélules molles ou dures.
Une préparation sous forme de sirop ou d'élixir peut par exemple contenir l'ingrédient actif (i) ou (ii) conjointement avec un édulcorant, un antiseptique, un conservateur, un agent donnant du goût ou un colorant approprié.
Les poudres, lyophilisats ou les granules dispersibles dans l'eau peuvent contenir l'ingrédient actif (i) ou (ii) en mélange avec des agents de dispersion ou des agents mouillants ou des agents de mise en suspension, de même qu'avec des correcteurs de goûts ou des édulcorants. A titre d'exemple, la composition (a) contenant au moins en tant qu'ingrédient actif (i), un inhibiteur de l'HMG CoA réductase pourra être sous forme de gélule, de capsule ou de comprimé et la composition (b) contenant au moins à titre d'ingrédients actif (ii), la citrulline pourra être sous forme de gélule, poudres, lyophilisats, granules, comprimé ou capsule mais aussi sous forme buvable, comme un sirop, une infusion, un jus, un macérât, cette liste n'étant en aucun cas limitative.
Selon un mode de réalisation préféré, les compositions (a) et (b) se présentent sous forme unitaire, de type gélule, comprimé, capsule ou sachet. Un tel sachet peut comprendre à titre d'exemple, de la poudre, un lyophilisat ou des granules. De façon particulièrement préférée, la composition (a) se présente sous forme de comprimé et la composition (b) se présente sous une forme unitaire de sachet.

Les produits de combinaison selon l'invention, sous forme adaptée à la prise par voie orale peuvent être également destinés à une utilisation en tant que complément alimentaire. Dans un tel mode de réalisation, les ingrédients actifs (i) et (ii) sous forme de composition unique ou de compositions distinctes (kit) sont dans un milieu adapté à la prise par voie orale. Les ingrédients actifs (i) et (ii) peuvent également être mélangés directement à un milieu alimentaire, un aliment ou à une boisson.
Lorsqu'un produit de combinaison selon l'invention se présente sous forme adaptée à la prise par voie orale en mélange avec un aliment ou une boisson, il pourra constituer un aliment fonctionnel. Par aliment fonctionnel on entend tout aliment ou boisson, par exemple, les produits issus de laits d'animaux (beurre, fromages, yaourts, laits) ou végétaux (laits, yaourts, céréales, jus de fruits ou de légumes, soupes, etc) auquel un produit de combinaison selon l'invention a été ajouté. De façon préférée, ledit produit de combinaison se trouve alors sous forme d'une composition unique ajoutée à un aliment tel que précédemment mentionné.
Un produit de combinaison, destiné à une utilisation en tant que complément alimentaire, ou un aliment fonctionnel tel que décrit ci-dessus pourra bien entendu comprendre également tout composé supplémentaire présentant un intérêt nutritionnel et/ou stimulant la perte de poids ou de masse grasse, comme des vitamines, des sels minéraux, des acides aminés essentiels, des acides gras essentiels, des oligoéléments, divers extraits végétaux, des fibres, des antioxydants, des flavonoïdes.
Lorsqu'un produit de combinaison selon l'invention est destiné à une utilisation en tant que complément alimentaire, il pourra également comprendre des composants, et en particulier les ingrédients actifs (i) et (ii), sous forme d'extraits végétaux les contenants, comme décrits précédemment.
L'invention concerne donc également un produit de combinaison comprenant comme seuls ingrédients à titre de substances actives :
(i) un inhibiteur de la HMG-CoA réductase sous forme d'extrait végétal en contenant, de préférence un extrait de levure rouge de riz, et/ou un extrait végétal contenant des monacolines et/ou de la berbérine et/ou des policosanols
(ii) la citrulline, de préférence sous forme d'extrait végétal en contenant,
lesdits ingrédients (i) et (ii) étant dans une forme adaptée à une administration par voie orale, séquentielle, séparée et/ou simultanée de l'un avec l'autre dans le temps.
Selon un mode de réalisation préféré, l'extrait végétal contenant de la citrulline est un extrait végétal obtenu à partir de plantes appartenant à la famille des cucurbitacées, en particulier un extrait végétal de pastèque et/ou de melon. Dans un autre mode de réalisation, le produit de combinaison comprend comme seuls ingrédients à titre de substances actives :
(i) un inhibiteur de la HMG-CoA réductase sous forme d'extrait végétal en contenant, de préférence un extrait de levure rouge de riz, et/ou un extrait végétal contenant des monacolines et/ou de la berbérine et/ou des policosanols
(ii) la citrulline, sous forme pure,
lesdits ingrédients (i) et (ii) étant dans une forme adaptée à une administration par voie orale, séquentielle, séparée et/ou simultanée de l'un avec l'autre dans le temps.
De préférence de tels produits de combinaison sont adaptés à une prise par voie orale, et de préférence encore ils sont destinés à une utilisation en tant que complément alimentaire.

La quantité d'ingrédients actifs (i) et (ii) des produits de l'invention ainsi que le dosage des dits produits dépendra de la finalité cosmétique ou thérapeutique de l'invention. En particulier dans le cas d'un traitement thérapeutique, cette quantité dépendra du trouble à traiter et de la sévérité de l'état du patient, du patient ou de l'individu lui-même, du mode d'administration ainsi que de la forme sélectionnée des composés (i) et (ii).
La dose administrée à un mammifère, être humain ou mammifère, dans le contexte de l'invention doit être suffisante pour induire un effet thérapeutique ou cosmétique dans une durée raisonnable. L'homme du métier pourra déterminer, la dose, la composition et le mode d'administration la plus appropriée d'un produit de combinaison selon l'invention. En particulier dans le cas d'un traitement pharmaceutique, ceux-ci pourront varier en fonction des propriétés pharmacologiques de la formulation, de la nature et de la sévérité du trouble à traiter (et/ou du surpoids ou de l'obésité), de la condition physique du patient ou de l'individu, de l'efficacité des ingrédients actifs (i) et (ii) sélectionnés ainsi que de l'âge, du poids, du sexe, et de la réponse du patient au traitement.
L'administration des ingrédients actifs (i) et (ii) selon l'invention peut être continue ou intermittente (par bolus). Le dosage peut donc être déterminé par les horaires et/ou la fréquence d'administration. Des doses adaptées d'ingrédients actifs incluent celles référencées dans la littérature médicale comme dans Martindale- The Complete Drug Reference (34th edition) ainsi que les documents qu'ils référencent. Par ailleurs, l'adaptation des dosages d'une espèce à l'autre est connue de l'homme du métier et peut être réalisée comme décrit dans Mordenti et al., Pharmarceut. Res. 8, p.1351-1358, 1991.
Par exemple des doses adaptées d'ingrédients actifs varient entre environ 0.01 mg/kg de masse corporelle et 1000 mg/kg de masse corporelle. De façon préférée, les gammes de dosage vont de 0.1 mg/kg à 20 mg/kg sur une base quotidienne et pour une administration orale.
A titre d'exemple, l'ingrédient actif (i) d'un produit de combinaison selon l'invention est administré à des doses journalières allant de 10 à 80 mg par jour et de préférence à une dose journalière allant de 30 à 50 mg, lorsqu'il s'agit de de statine de synthèse, par exemple l'atorvastatine, la simvastatine et la pravastatine.
Lorsque l'ingrédient actif (i) est une statine naturelle, sous forme d'un extrait standardisé de levure rouge de riz en contenant, ledit extrait est généralement administré à des doses de 100 à 600 mg de une à trois fois par jour. De préférence un tel extrait standardisé comprend entre 1 et 4 % de monacoline K, plus particulièrement entre 1 et 3 % et typiquement un tel extrait contient 2 % de monacoline K.
La citrulline, est typiquement administrée à une dose quotidienne allant de 1 à 15 g/jour et de préférence à une dose de 2 à 5 g/jour. Ces doses pourront être administrées en une prise ou sous forme fractionnée, par exemple en deux à trois prises.
L'homme du métier adaptera les compositions de l'invention en fonction de la forme galénique et du nombre de prises adaptées à l'administration de la dose d'ingrédient actif désirée.
Ainsi lorsque les compositions de l'invention se présentent sous forme unitaire comme décrit précédemment, elles pourront contenir des doses de citrulline allant de 50 à 4000 mg, en particulier de 100 à 3000 mg, en particulier de 200 à 1000 mg. Les formes unitaires pourront également comprendre des doses de statines allant de 2 à 100 mg, de 5 à 80 mg ou encore de 10 à 40 mg. Enfin, la levure rouge de riz pourra être présente dans des formes unitaires de l'invention à des doses allant de 30 à 1000 mg, de 50 à 800 mg ou de 100 à 600 mg.
Par exemple, lorsque le produit de combinaison se présente sous la forme d'une composition unique, ladite composition pourra être administrée par voie orale en continu par perfusion intraveineuse. Une telle administration pourra avoir lieu pendant des durées variable dépendantes du patient et de son état. L'administration pourra être interrompue puis renouvelée de 1 à plusieurs fois. De façon préférée cependant, une telle composition est adaptée à la prise par voie orale.
Lorsque le produit de combinaison se présente sous forme d'une trousse contenant au moins une composition (a) et une composition (b) distinctes, telles que précédemment décrites, il est entendu que les deux compositions pourront être administrées selon par des voies identiques ou similaires. Par exemple une composition pourrait être administrée par voie injection, par exemple en continue par perfusion et une autre composition du produit de combinaison pourrait être administrée par voie entérale, en particulier orale, par bolus ou de façon fractionnée en plusieurs prises, au cours de la journée. Les deux compositions peuvent également être administrées par voie entérale, en particulier par voie orale, selon des fréquences différentes ou identiques. De façon préférée les compositions (a) et (b) sont administrées par voie orale en une ou plusieurs prises quotidiennes. Une composition pouvant être prise par bolus quotidiennement, par exemple le matin ou le soir, et l'autre composition de façon fractionnée en plusieurs prises quotidiennes, par exemple, matin et soir ou encore, matin, midi et soir. Lorsque les ingrédients actifs (i) et (ii) sont présents dans deux compositions séparées (a) et (b), il est possible de prendre les dits ingrédients selon des fréquences ou des durées différentes ou encore de les prendre séquentiellement (une cure après l'autre ou simplement à des moments différents de la journée). Par exemple l'ingrédient (i) peut être pris une fois par jour alors que l'ingrédient (ii) pourrait être pris 3 fois par jour. La prise de l'ingrédient (ii) pourra également être prolongée après l'arrêt de la cure de l'ingrédient (i).
L'administration d'un produit de combinaison tel que décrit dans la présente invention pourra donc être réalisée de façon répétée, sous forme de cure, pendant plusieurs jours ou plusieurs semaines (par exemple 4 semaines). Eventuellement, plusieurs cures successives peuvent être entreprises par exemple 2 à 4, éventuellement interrompues par une pause de 1 semaine à 3 mois. Dans le cadre d'un traitement préventif, l'administration d'un produit de combinaison selon l'invention peut être réalisée de façon chronique.

L'invention porte enfin sur un procédé ou une méthode de traitement cosmétique pour améliorer l'apparence d'un individu, par exemple pour affiner la silhouette et/ou diminuer ou limiter les amas graisseux localisés ou lipodystrophie, et/ou stimuler la perte de poids superflu et/ou de cellulite, et/ou limiter son accumulation, de préférence sans perte de masse maigre chez les individus en bonne santé.
Par poids superflu on entend que ledit procédé cosmétique est destiné aux sujets (l'homme ou l'animal, par exemple les animaux de compagnie, d'élevage, de course ou de concours) de corpulence normale. Chez les êtres humains par exemple, on considérera les sujets en bonne santé, dont le surpoids ou la surcharge graisseuse n'est pas associée à des états pathologiques, ou à une augmentation de la charge de morbidité (typiquement des troubles du métabolisme du glucose, une résistance à l'insuline, un syndrome métabolique, un diabète ou des troubles vasculaires) et dont l'IMC est compris entre 18,5 et 25. Chez de tels sujets le poids ou la masse grasse typiquement sous forme de cellulite, n'est pas associée à une augmentation de la charge de morbidité et est préférentiellement répartie selon une morphologie gynoïde. Un traitement cosmétique selon l'invention sera ainsi particulièrement adapté au traitement de la cellulite, en particulier sur les hanches, et les fesses. Il pourra être utilisé par exemple chez les femmes en période de ménopause ou après la grossesse et de façon générale chez des individus ayant un mode de vie sédentaire et/ou une alimentation riche en sucre ou en graisse, afin d'aider à conserver ou retrouver une silhouette mince et à éliminer ou prévenir l'apparition de cellulite.
Un tel traitement peut également être utile chez les animaux castrés, afin de prévenir l'augmentation de la masse grasse au détriment de la masse maigre musculaire.

La perte de poids est souvent induite par des régimes restrictifs, difficiles à respecter sur le long terme, souvent responsables d'un effet rebond (ou rechute) entraînant une reprise de poids parfois supérieure à celle induite par la restriction calorique, un procédé de traitement cosmétique selon l'invention pourra donc être utilisé en complément d'un régime alimentaire restrictif et éventuellement d'une augmentation de l'activité physique, afin de limiter l'intensité de la restriction notamment calorique et de prévenir la perte de masse maigre et l'effet rebond lié à l'arrêt du régime restrictif.

Un procédé de traitement cosmétique selon l'invention comprend l'administration d'au moins (i) un inhibiteur de l'HMG-CoA réductase et (ii) la citrulline, en particulier sous forme d'un produit de combinaison tel que précédemment défini. Un tel produit de combinaison peut être sous forme de composition unique ou sous forme de trousse, comme précédemment décrit. Dans un tel procédé l'administration d'au moins (i) un inhibiteur de l'HMG-CoA réductase et (ii) la citrulline et/ou l'un de ses sels pourra être réalisée de façon simultanée sous forme de composition unique ou de compositions distinctes. Lorsque les compositions sont distinctes, elles pourront également être administrées de façon séparées et/ou séquencées dans le temps, selon les modalités définies précédemment.
De façon particulièrement préférée, le produit de combinaison est adapté à la prise par voie orale et est destiné à une prise sous forme de complément alimentaire comme précédemment décrit. Ledit produit pourra donc bien entendu comprendre tout composé supplémentaire présentant un intérêt nutritionnel et/ou stimulant la perte de poids ou de masse grasse, comme décrit précédemment ; la présence de l'arginine dans un produit de combinaison selon l'invention est exclue. Le produit de combinaison pourra comprendre, à titre d'exemple, des vitamines, des sels minéraux, des acides aminés essentiels, des acides gras essentiels, des oligoéléments, divers extraits végétaux, des fibres, des antioxydants, des flavonoïdes. On peut également citer les composants naturels aux propriétés anorexigène, comme des extraits d'hoodia, d'eucalyptus, de laurier sauce, de coca, de catharanthus, de phyllantus niruri, d'orthosiphon, d'algues, de caroube, de konjac, brûle-graisse (ou augmentant la thermogénèse), comme des extraits de fucus, d'algues marines, de thé vert, de maté, de guarana, de coleus, de garcinia de caféine, dépuratives, comme des extraits de pissenlit, artichaut, romarin, chardon marie, curcuma, hercampuri (gentianella) ou calmantes pour diminuer le stress souvent en cause dans l'obésité et permettre de mieux supporter le régime hypocalorique, comme des extraits de millepertuis (Hypericum), pavot de Californie (Eschscholtzia), valériane (Valeriana), tilleul (Tilia).
Les ingrédients (i) et (ii) sont les seuls ingrédients administrés à titre de substance active pour affiner la silhouette et/ou diminuer ou limiter les amas graisseux localisés ou lipodystrophie, et/ou stimuler la perte de poids superflu et/ou de cellulite, et/ou limiter son accumulation.

Par ailleurs, un tel produit de combinaison comprendra de façon préférée des composants, et en particulier les ingrédients actifs (i) et (ii), d'origine naturelle, typiquement sous forme d'extraits végétaux. Par exemple le produit de combinaison pourra comprendre (i) un extrait de levure rouge de riz, et/ou un extrait végétal contenant du policosanol, et/ou de la berbérine et/ou des monacolines et (ii) un extrait végétal obtenu à partir d'une plantes appartenant à la famille des cucurbitacés, en particulier un extrait végétal de pastèque et/ou de melon.
Chez l'homme, un extrait standardisé de levure rouge riz est généralement administré à des doses allant de 100 à 600 mg de une à trois fois par jour.
La citrulline est généralement administrée chez l'homme à des doses comprises allant de 1 à 15 g/jour en particulier des à des doses allant de 2 à 5 g/jour.

La présente invention est illustrée à titre non limitatif par les exemples ci-après. Dans ces exemples, on se référera aux figures suivantes :
- Figure 1.: Schéma du déroulement temporel des expériences pharmaco-nutritionnelle. (HL) : régime hyperlipidique, (HL+cit) : régime hyperlipidique supplémenté en citrulline seule, (HL+cit+stat) : régime hyperlipidique supplémenté en citrulline et atorvastatine, (HL+stat) : régime hyperlipidique supplémenté en atorvastatine seule
- Figure 2.: Effet des régimes : contrôle, HL, cit+stat (HL+cit+stat), stat (HL+stat), cit (HL+cit), sur le poids des souris. a vs. b et c vs. B : p < 0.05 ; a vs. c = 0.06.
- Figure 3.: Effet des régimes : contrôle, (HL), (HL+cit), (HL+cit+stat) et (HL+stat), sur la masse maigre (Fig. 3A) et la masse grasse (Fig. 3B) des souris évaluées par EchoMRI.
- Figure 4.: Effet des régimes : contrôle, (HL), (HL+cit), (HL+cit+stat) et (HL+stat), sur les poids du tissu adipeux (Fig. 4A), de muscles de la patte (Fig. 4B) et du foie (Fig. 4C).
- Figure 5.: Effet des régimes : contrôle (◆), HL (■), HL+cit (**x**), HL+cit+stat (▪) et HL+stat (▲), sur la tolérance au glucose (Fig. 5A : glycémie (mg/dl), Fig. 5B : Glucose AUC (i.e. : aire sous la courbe,Unité Arbitraire) et la sensibilité à l'insuline (Fig. 5C : glycémie (mg/dl), contrôle (◆), HL (▪), HL+cit (**x**), HL+cit+stat (**x**) et HL+stat (▲)), Fig. 5D : diminution de la glycémie (UA)).

### EXEMPLES :

### 1. Matériels et méthodes

### 1.1 Les animaux :

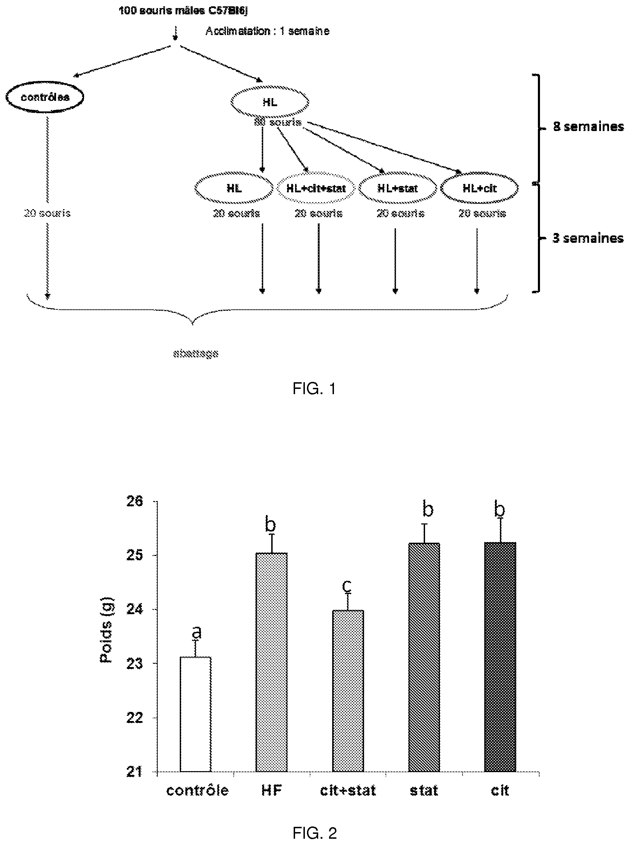

100 souris mâles C57 Black 6/J de 5 semaines (Janvier, France) ont été hébergées en cages individuelles à l'animalerie du centre INRA de Theix, dans un local thermorégulé à 20-22°C et soumis à une alternance de cycles de lumière et d'obscurité de 12h. Après une semaine d'acclimatation sous régime « contrôle » (D12450B, Research Diet, USA), 20 souris ont poursuivi ce régime durant 11 semaines. Les 80 autres souris ont été nourries avec un régime « hyperlipidique » (HL) (D12451, Research Diet, USA) durant 8 semaines.
En pourcentage des apports caloriques totaux, les régimes « contrôle » et HL apportaient tous les deux 20% de protéines et respectivement, 70% et 35% de glucides et 10% et 45% de lipides. Après huit semaines de régime hyperlipidique, les 80 souris ont été réparties en 4 groupes de 20 animaux et soumises respectivement à un régime hyperlipidique seul (HL), enrichi en L-Citrulline seule (2,5g/kg) (HL+cit), en Atorvastatine seule (10mg/kg) (HL+stat) ou en L-Citrulline (2,5g/kg) et en Atorvastatine (10mg/kg) (HL+cit+stat), pendant trois semaines.
Les animaux avaient libre accès aux régimes et à l'eau durant l'intégralité de l'étude (figure 1). Le poids des souris était mesuré toutes les 2 semaines.

### 1.2 Mesure de la sensibilité à l'insuline et de la tolérance au glucose

Après 3 semaines d'intervention pharmaco-nutritionnelle, des mesures de sensibilité à l'insuline et de tolérance au glucose ont été réalisées respectivement sur 10 souris de chaque groupe après une nuit de jeûne.

Mesure de la sensibilité à l'insuline : la glycémie basale a été évaluée à l'aide d'un glucomètre (Glucotouch, Lifescan, USA) sur une goutte de sang prélevée à la queue. Une dose d'insuline de 0,75 mU/g de poids était injectée par voie intra-péritonéale et la glycémie était évaluée 15, 30, 45, 60 et 120 minutes après l'injection.

Mesure de la tolérance au glucose : une dose de glucose (2 mg/g de poids) était injectée par voie intrapéritonéale. La glycémie a été mesurée comme pour le test de sensibilité à l'insuline avant et 15, 30, 60 et 120 minutes après l'injection.

### 1.3 Mesures de composition corporelle par ECHO MRI

La masse grasse et la masse maigre des souris vigiles et à jeun depuis 15h ont été quantifiées par Résonance Magnétique Nucléaire (Echo MRI, Houston, USA) au 80ème jour de l'intervention.

### 1.4 Prélèvement des organes et des tissus

Avant le sacrifice, les souris à jeun depuis 15h ont été anesthésiées par une injection intrapéritonéale de pentobarbital (1,5 µl/g). Le sang a été recueilli dans des tubes contenant de l'EDTA avant d'être centrifugé (12000 rpm, 4 min, température ambiante). Le plasma était ensuite prélevé, réparti en fractions aliquotes et congelé dans l'azote liquide puis conservé à - 80°C. Le tissu adipeux péri-épididymaire, le foie et les muscles gastrocnémien, *tibialis* et quadriceps étaient pesés, congelés dans l'azote liquide puis conservés à -80°C.

### 1.5 Dosages plasmatiques

Les concentrations plasmatiques d'acides gras non estérifiés (AGNE) (NEFA-HR(2) R1 et R2 434-91795/436-91995, Wako Chemicals GmbH), de triglycérides (TG) (981786, Thermo Fischer Scientific), de cholestérol (981813 Thermo Fischer Scientific), et de glucose (Glucose God-Pod, 981780 Thermo Fischer Scientific) ont été mesurées à l'aide de l'automate Konelab 20 (Thermo Electron Corporation, France).

### 1.6 Analyses statistiques

Les résultats sont présentés sous la forme moyenne ± erreur standard. Les résultats ont été analysés par analyse de variance (ANOVA) et test post-hoc de Fischer en post-hoc à l'aide du logiciel Statview (SAS Institute, USA). Le niveau de significativité a été fixé à p < 0.05.

### 2. Résultats

### 2.1 Evolution du poids des animaux

Après 3 semaines d'intervention pharmaco-nutritionnelle, les poids des souris des différents groupes étaient significativement différents (Fig. 2, ANOVA, p<0,0001). Les poids des souris soumises aux régimes HL, HL+stat et HL+cit étaient significativement plus élevés par rapport aux contrôles (respectivement, p<0,001, p<0,0001, p<0,0001). Seul le régime HL+cit+stat a permis de diminuer significativement le poids des souris par rapport aux autres souris soumises aux régimes HL, HL+cit ou HL+stat (P<0.05) et à un niveau approchant celui des contrôles (p=0.06).

### 2.2 Analyse de la composition corporelle des animaux

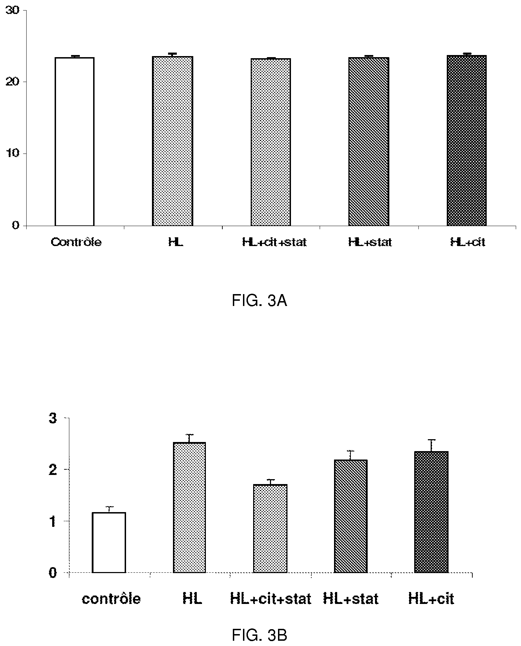

Analyse par Echo MRI : les masses maigres des souris des 5 lots n'étaient pas significativement différentes (Fig. 3A, ANOVA, p=NS). En revanche, la masse grasse totale des souris soumises aux régimes HL, HL+cit, HL+stat et HL+cit+stat était significativement plus élevée (respectivement +115%, +100%, +87%, +46%) que celes des souris ayant suivant le régime contrôle (ANOVA, p<0,0001). Néanmoins, la masse grasse totale des souris soumises au régime HL+cit+stat était plus faible que celle des régimes HL (-32%), HL+cit (-27%) et HL+stat (-22%)(P=0.06) (figure 3B).

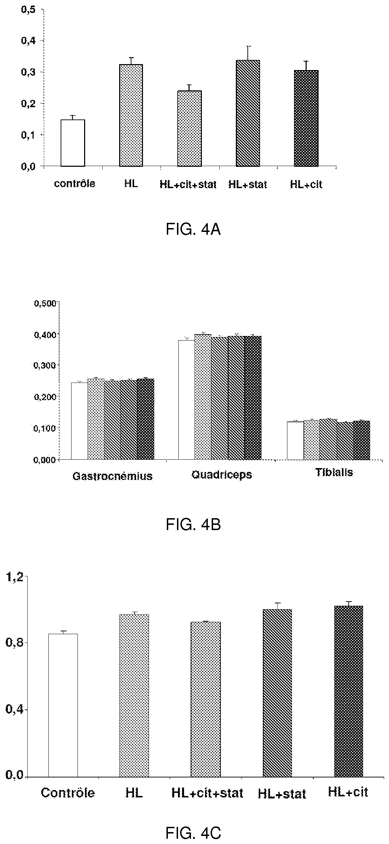

Par pesée des tissus au sacrifice : chez les souris ayant suivi les régimes HL, HL+cit, HL+stat, le poids du tissu adipeux péri-épididymaire était doublé par rapport aux souris sous régime contrôle (Fig. 4A, ANOVA, p<0,005). Le poids du tissu adipeux péri-épididymaire des souris ayant reçu le régime HL+cit+stat était plus faible par rapport à celui des autres lots HL (-26% vs. HL, -22% vs. HL+citr, -30% vs. HL+stat)(Fig. 4A). L'absence de différence significative entre les masses maigres des souris évaluées par Echo MRI a été confirmée par pesée des organes. La somme des poids des muscles gastrocnémien, *tibialis* et quadriceps n'était pas significativement différente entre les groupes (Fig.4B). Le poids du foie ne variait pas non plus significativement (Fig. 4C).

### 2.3 Paramètres plasmatiques

Les concentrations plasmatiques en AGNE (acides gras non estérifiés), TG (triglycérides), cholestérol et glucose des différents groupes, sont présentées dans le tableau 1. La concentration plasmatique en AGNE chez les souris soumises au régime HL était significativement supérieure en moyenne de 30% à celle des autres groupes (ANOVA, p<0,05). Les régimes HL+cit, HL+cit+stat et, dans une moindre mesure, HL+stat, ont permis de rétablir la concentration plasmatique en AGNE au niveau de celui des souris contrôles. De même, la concentration plasmatique en TG était significativement plus haute dans le groupe des souris soumises au régime HL par rapport aux souris ayant reçu les autres régimes (ANOVA, p<0,05). Les régimes HL+cit, HL+stat et HL+cit+stat ont permis de rétablir la triglycéridémie au niveau de celle obtenue chez les souris contrôles. Les régimes HL, HL+cit, HL+stat et HL+cit+stat ont induit une augmentation significative de la cholestérolémie par rapport à celle des souris contrôles (ANOVA, p=0,01). Aucune différence significative de la glycémie n'a été mise en évidence entre les 5 groupes (tableau 1).

### 2.4 Sensibilité à l'insuline et tolérance au glucose

Mesure de la tolérance au glucose (test ipGTT) : la tolérance au glucose se définit comme la capacité d'une souris à normaliser sa glycémie lors d'une injection de glucose. Elle est estimée par le calcul de l'aire sous la courbe de l'évolution de la glycémie suite à l'injection de glucose.

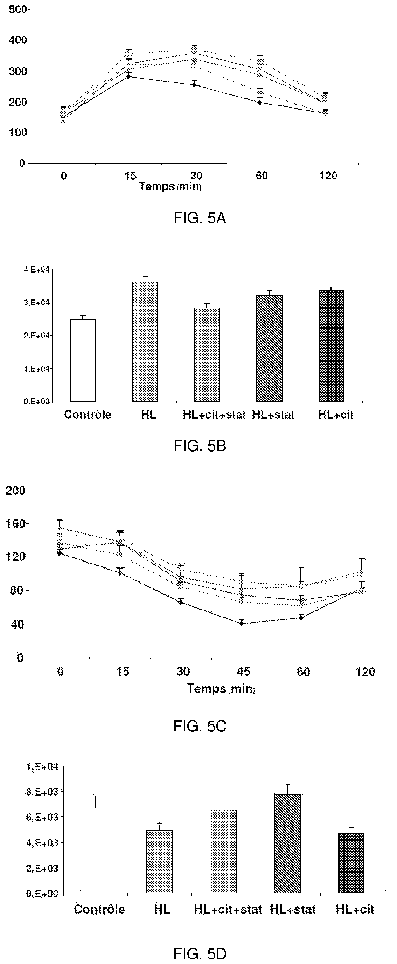

Les régimes HL, HL+cit, HL+stat ont entraîné une diminution significative de la tolérance au glucose par rapport au régime contrôle (respectivement p<0,0001 ; p=0,0001 et p=0,0008). Seul le régime HL+cit+stat a permis de rétablir la tolérance au glucose à hauteur de celle des souris contrôles. La réponse glycémique de ce groupe HL+cit+stat est améliorée par rapport à celle du groupe HL+stat (p=0,07) et est significativement différente de celles des groupes HL+cit et HL (respectivement p<0,05 et p<0,001) (fig. 5A et 5B).

Mesure de la sensibilité à l'insuline (test ipITT) : de la même manière, les souris sous régime HL ou HL+cit tendent à être moins sensibles à l'insuline par rapport aux contrôles (respectivement, p=0,10 p=0,07) et au groupe HL+stat (respectivement p<0,05 et p<0,01). Le régime HL+cit+stat a permis de maintenir la sensibilité à l'insuline à un niveau comparable à celui des souris contrôles (fig. 5C et 5D).

### 3. Conclusion

L'association de la L-citrulline (végétale ou de synthèse) et d'une statine (végétale ou de synthèse) permet de limiter la prise de poids et entraîne un effet synergique puissant sur la prise de masse grasse, sous régime obésogène.
En outre, une telle association possède également un effet synergique permettant de prévenir l'intolérance au glucose et l'insulino-résistance associées à la prise de poids excessive.
Le tableau 1 ci-dessous récapitule les actions synergiques entre L-citrulline et statine pour la prévention de la prise de poids et du syndrome métabolique induits par un régime hyperlipidique :

**Tableau 1 :**

| | **L-citrulline** | **Statine** | **L-citrulline + statine** |
|---|---|---|---|
| Poids et composition corporelle | | | |
| Poids corporel | - | - | **-4%** |
| Masse grasse | -7% | -13 % | **-32 %** |

| Concentrations plasmatiques | | | |
|---|---|---|---|
| Glucose | - | - | - |
| Triglycérides | -15 % | -13 % | -15 % |
| Acides gras non estérifiés | -24 % | -11 % | -17 % |
| Cholestérol* | - | - | - |

| Homéostasie glucidique | | | |
|---|---|---|---|
| Tolérance au glucose | +7 % | +11 % | **+21 %** |
| Sensibilité à l'insuline | +5 % | +31 % | **+34 %** |

| | | | |
|---|---|---|---|
| *Les pourcentages expriment les différences avec le régime hyperlipidique seul (HL);* "-" *signifie que l'effet est non significatif.* ** la souris n'est pas un bon modèle pour explorer la réponse LDL*/*HDL* | | | |

## Revendications

1. Produit de combinaison comprenant à titre de substances actives, au moins :
i. un inhibiteur de l'enzyme HMG-CoA-réductase, et
ii. la citrulline ou l'un de ses sels ;
pour utilisation dans le traitement du surpoids, de l'obésité ou de la surcharge de masse grasse ;
ledit produit ne comprenant pas d'arginine et la citrulline étant administrée à une dose quotidienne de 1 à 15 g/jour.

2. Produit de combinaison pour utilisation selon la revendication 1, **caractérisé en ce que** les substances actives consistent en :
i. au moins un inhibiteur de l'enzyme HMG-CoA-réductase, et
ii. la citrulline.

3. Produit de combinaison pour utilisation selon l'une des revendications 1 ou 2, **caractérisé en ce qu'**il est sous la forme d'une composition unique contenant ledit inhibiteur de l'enzyme HMG-CoA-réductase et ladite citrulline, dans un milieu physiologiquement acceptable.

4. Produit de combinaison pour utilisation selon l'une des revendications 1 ou 2, **caractérisé en ce qu'**il est sous forme d'une trousse contenant :
(a) une composition incluant ledit inhibiteur de l'enzyme HMG-CoA-réductase, dans un milieu physiologiquement acceptable ; et
(b) une composition incluant ladite citrulline dans un milieu physiologiquement acceptable ;
lesdites compositions (a) et (b) étant chacune fournies sous une forme qui convient à une administration séquentielle, séparée et/ou simultanée de l'une avec l'autre dans le temps.

5. Produit de combinaison pour utilisation selon l'une au moins des revendications 1 à 4, **caractérisé en ce que** les ingrédients actifs (i) et (ii) ou les compositions (a) et (b) sont dans un milieu adapté à l'administration par voie orale.

6. Produit de combinaison pour utilisation selon l'une au moins des revendications 1 à 5, ledit produit étant destiné à des sujets présentant une intolérance au glucose.

7. Produit de combinaison pour utilisation selon l'une au moins des revendications précédentes, **caractérisé en ce que** la citrulline est sous forme d'un extrait de plante appartenant à la famille des *cucurbitaceae.*

8. Produit de combinaison pour utilisation selon l'une au moins des revendications précédentes, **caractérisé en ce que** l'inhibiteur de la HMG-CoA réductase est choisi parmi le groupe constitué par les statines d'origines naturelles ou synthétiques, les monacolines, la berbérine, et le policosanol.

9. Produit de combinaison pour utilisation selon l'une au moins des revendications 1 à 8, **caractérisé en ce que** l'inhibiteur de la HMG-CoA réductase se présente sous la forme d'un extrait de levure rouge de riz, ou *monascus purpureus.*

10. Produit de combinaison pour utilisation selon l'une au moins des revendications précédentes, destiné à des sujets stressés ou angoissés, et/ou ayant une alimentation riche en graisses ou en sucre, et/ou un mode de vie sédentaire, et/ou présentant des dérèglements hormonaux et/ou une susceptibilité familiale au surpoids ou à l'obésité, ou encore prenant des médicaments entraînant une prise de poids.

11. Produit de combinaison pour utilisation selon l'une quelconque des revendications 1 à 10, **caractérisé en ce que** l'ingrédient actif (i) est administré à une dose quotidienne de 10 à 80 mg/jour, lorsqu'il s'agit de statine synthétique et l'ingrédient actif (ii) est administré à une dose quotidienne de 2 à 5 g/jour.

12. Produit de combinaison comprenant comme seuls ingrédients à titre de substances actives :
(i) un inhibiteur de la HMG-CoA réductase sous forme d'extrait végétal en contenant, de préférence un extrait de levure rouge de riz, et/ou un extrait végétal contenant des monacolines et/ou de la berbérine et/ou des policosanols,
(ii) la citrulline ou l'un de ses sels, de préférence sous forme d'extrait végétal en contenant ;
lesdits ingrédients (i) et (ii) étant dans une forme adaptée à une administration par voie orale, séquentielle, séparée et/ou simultanée de l'un avec l'autre dans le temps.

13. Procédé de traitement cosmétique pour améliorer ou affiner la silhouette et/ou stimuler la perte de poids superflu et/ou de cellulite et/ou limiter son accumulation, comprenant l'administration d'un produit de combinaison tel que défini dans la revendication 12, et le renouvellement de ladite administration jusqu'à obtention de l'effet cosmétique désiré.

14. Procédé de traitement cosmétique selon la revendication 13, **caractérisé en ce que** l'ingrédient actif (i) est administré à une dose comprise entre 100 et 600 mg de une à trois fois par jour et la citrulline est administrée à une dose quotidienne comprise entre 2 et 5 g/jour.

15. Utilisation d'un produit, selon la revendication 12, pour améliorer ou affiner la silhouette et/ou stimuler la perte de poids superflu et/ou de cellulite et/ou limiter son accumulation.

## Patentansprüche

1. Kombinationspräparat, enthaltend als Wirkstoffe mindestens
i. einen Inhibitor des Enzyms HMG-CoA-Reduktase, und
ii. Citrullin oder eines seiner Salze;
zur Verwendung bei der Behandlung von Übergewicht, Fettleibigkeit oder übermäßiger Fettmasse;
wobei das Produkt kein Arginin umfasst und das Citrullin in einer täglichen Dosis von 1 bis 15 g/Tag verabreicht wird.

2. Kombinationspräparat für die Verwendung nach Anspruch 1, **dadurch gekennzeichnet, dass** die Wirkstoffe bestehen aus:
i. mindestens einem Inhibitor des Enzyms HMG-CoA-Reduktase, und
ii. Citrullin.

3. Kombinationspräparat für die Verwendung nach einem der Ansprüche 1 oder 2, **dadurch gekennzeichnet, dass** es in Form einer Einzelzusammensetzung vorliegt, enthaltend den Inhibitor des Enzyms HMG-CoA-Reduktase und das Citrullin in einem physiologisch akzeptablen Medium.

4. Kombinationspräparat für die Verwendung nach einem der Ansprüche 1 oder 2, **dadurch gekennzeichnet, dass** es in Form eines Kits vorliegt, enthaltend:
(a) eine Zusammensetzung, die den Inhibitor des Enzyms HMG-CoA-Reduktase in einem physiologisch akzeptablen Medium einschließt; und
(b) eine Zusammensetzung, die das Citrullin in einem physiologisch akzeptablen Medium einschließt;
wobei die Zusammensetzungen (a) und (b) jeweils in einer Form bereitgestellt werden, die für eine sequentielle, getrennte und/oder miteinander gleichzeitige Verabreichung über die Zeit geeignet ist.

5. Kombinationspräparat für die Verwendung nach mindestens einem der Ansprüche 1 bis 4, **dadurch gekennzeichnet, dass** die Wirkstoffe (i) und (ii) oder die Zusammensetzungen (a) und (b) in einem für die orale Verabreichung angepassten Medium vorliegen.

6. Kombinationspräparat für die Verwendung nach mindestens einem der Ansprüche 1 bis 5, wobei das Produkt für Subjekte mit Glukoseintoleranz bestimmt ist.

7. Kombinationspräparat für die Verwendung nach mindestens einem der vorangehenden Ansprüche, **dadurch gekennzeichnet, dass** das Citrullin in der Form eines Extrakts von Pflanzen aus der Familie *Cucurbitaceae* vorliegt.

8. Kombinationspräparat für die Verwendung nach mindestens einem der vorangehenden Ansprüche, **dadurch gekennzeichnet, dass** der HMG-CoA-Reduktase-Inhibitor aus der Gruppe ausgewählt ist, die aus Statinen natürlichen oder synthetischen Ursprungs, Monacolinen, Berberin und Policosanol besteht.

9. Kombinationspräparat für die Verwendung nach mindestens einem der Ansprüche 1 bis 8, **dadurch gekennzeichnet, dass** der HMG-CoA-Reduktase-Inhibitor in Form eines Extraktes aus roter Reishefe, oder *Monascus purpureus,* vorliegt.

10. Kombinationspräparat für die Verwendung nach mindestens einem der vorangehenden Ansprüche, zur Behandlung von Subjekten, die unter Stress oder Angst leiden und/oder eine fett- oder zuckerreiche Ernährung haben und/oder eine sitzende Lebensweise aufweisen und/oder hormonelle Ungleichgewichte und/oder familiäre Anfälligkeit für Übergewicht oder Fettleibigkeit aufweisen oder des Weiteren Medikamente einnehmen, die zu einer Gewichtszunahme führen.

11. Kombinationspräparat für die Verwendung nach einem beliebigen der Ansprüche 1 bis 10, **dadurch gekennzeichnet, dass** der Wirkstoff (i) bei einer täglichen Dosis von 10 bis 80 mg/Tag, im Falle der Verabreichung eines synthetischen Statins, verabreicht wird, und der Wirkstoff (ii) bei einer täglichen Dosis von 2 bis 5 g/Tag verabreicht wird.

12. Kombinationspräparat, das als einzige Bestandteile die folgenden Wirkstoffe umfasst:
(i) einen HMG-CoA-Reduktase-Inhibtor in Form eines Pflanzenextrakts, der vorzugsweise einen Extrakt von roter Reishefe enthält, und/oder eines Pflanzenextrakts, der Monacoline und/oder Berberin und/oder Policosanole enthält,
(ii) Citrullin oder eines seiner Salze, vorzugsweise in Form eines Pflanzenextrakts, der es enthält; wobei die Bestandteile (i) und (ii) in einer Form vorliegen, die für eine sequentielle, getrennte und/oder miteinander gleichzeitige Verabreichung über die Zeit auf oralem Wege angepasst ist.

13. Kosmetisches Behandlungsverfahren zum Verbessern oder Schlankmachen der Figur und/oder zum Stimulieren des Abbaus von Übergewicht und/oder Zellulitis und/oder zum Begrenzen der Ansammlung derselben, umfassend die Verabreichung eines Kombinationspräparats, wie in Anspruch 12 definiert, und die Wiederholung der Verabreichung, bis der gewünschte kosmetische Effekt erreicht ist.

14. Kosmetisches Behandlungsverfahren nach Anspruch 13, **dadurch gekennzeichnet, dass** der Wirkstoff (i) bei einer Dosis zwischen 100 und 600 mg ein- bis dreimal täglich verabreicht wird, und das Citrullin bei einer täglichen Dosis zwischen 2 und 5 g/Tag verabreicht wird.

15. Verwendung eines Produkts nach Anspruch 12, um die Figur zu verbessern oder schlank zu machen und/oder den Abbau von Übergewicht und/oder Zellulitis zu stimulieren und/oder die Ansammlung derselben zu begrenzen.

## Claims

1. A combination product including as active substances, at least:
i. an inhibitor of the HMG-CoA-reductase enzyme, and
ii. citrulline or one of its salts;
for use in the treatment of excess weight or obesity, or excess body fat;
said product does not comprise any arginine and citrulline is administered at a daily dose of from 1 to 15 g/day.

2. A combination product for use according to claim 1, **characterized in that** the active substances consist in:
i. at least an inhibitor of the HMG-CoA-reductase enzyme, and
ii. citrulline.

3. A combination product for use according to claim 1 or 2, **characterized in that** it comes as a single composition including said inhibitor of the HMG-CoA-reductase enzyme and citrulline, in a physiologically acceptable medium.

4. A combination product for use according to claim 1 or 2, **characterized in that** it comes as a kit including:
(a) a composition comprising said inhibitor of the HMG-CoA-reductase enzyme, in a physiologically acceptable medium; and
(b) a composition comprising said citrulline in a physiologically acceptable medium;
said compositions (a) and (b) each being provided in a form which is suitable to a sequential, a separate and/or a simultaneous administration with one another over time.

5. A combination product for use according to anyone of claims 1 to 4, **characterized in that** the active substances (i) and (ii) or the compositions (a) and (b) are in a medium adapted to oral administration.

6. A combination product for use according to anyone of claims 1 to 5, said product being aimed at subjects suffering from glucose intolerance.

7. A combination product for use according to any preceding claim, **characterized in that** citrulline comes as a plant extract belonging to the *cucurbitaceae* plant family.

8. A combination product for use according to any preceding claim, **characterized in that** the HMG-CoA reductase inhibitor is selected from the group consisting of statins of natural or synthetic origin, monacolins, berberin, and policosanol.

9. A combination product for use according to anyone of claims 1 to 8, **characterized in that** the HMG-CoA reductase inhibitor comes as a red yeast rice extract, or *monascus purpureus.*

10. A combination product for use according to any preceding claim, for the treatment of stressed or anxious subjects, and/or subjects having a diet with a high fat or a high sugar content, and/or a sedentary lifestyle, and/or suffering from hormonal imbalances and/or from a genetic susceptibility to excess weight and/or obesity, or taking prescription drugs which can cause weight gain.

11. A combination product for use according to anyone of claims 1 to 10, **characterized in that** the active substance (i) is administered at a daily dosage of from 10 to 80 mg/day, when using a synthetic statin and the active substance (ii) is administered at a daily dosage of from 2 to 5 g/day.

12. A combination product comprising as sole active substances:
(i) a HMG-CoA reductase inhibitor in the form of a plant extract containing the same, preferably a red yeast rice extract, and/or a plant extract including monacolins and/or berberin and/or policosanols;
(ii) citrulline or one of the salts thereof, preferably in the form of a plant extract containing the same;
said substances (i) and (ii) being in a form adapted to a sequential, a separate and/or a simultaneous oral administration with one another over time.

13. A cosmetic treatment method for improving or slimming the figure and/or for stimulating the loss of excess weight and/or of cellulite and/or for limiting the localized accumulation thereof, comprising the administration of a combination product such as defined in claim 12, and the renewal of said administration until the expected cosmetic effect is obtained.

14. A cosmetic treatment method according to claim 13, **characterized in that** the active substance (i) is administered at a dose between 100 and 600 mg one to three times a day and citrulline is administered at a daily dose between 2 and 5 g/day.

15. Use of a product according to claim 12, for improving or slimming the figure and/or for stimulating the loss of excess weight and/or of cellulite and/or for limiting the accumulation thereof.
